# EUROPÄISCHE PATENTANMELDUNG

(11) **EP 2 033 626 A2**
(43) Veröffentlichungstag der Anmeldung: **11.03.2009**
(21) Anmeldenummer: 08162734.1
(22) Anmeldetag: 21.08.2008
(51) Int. Cl.: A61K 8/81, A61Q 5/06, C08F 26/06, C08F 26/10, C11D 3/37

(54) **Imidazoliumalkyl(meth)acrylat-Polymere**

(30) Priorität: 05.09.2007 EP 07115757
(71) Anmelder: BASF SE, 67056 Ludwigshafen (DE)
(72) Erfinder: Garcia Castro, Ivette, 67067 Ludwigshafen (DE); Dieckmann, Yvonne, 67454 Haßloch (DE); Haupt, Ingrid, 67227 Frankenthal (DE)

(57) **Zusammenfassung**

Die vorliegende Erfindung betrifft die Verwendung von Imidazoliumalkyl(meth)acrylat-Homo- und Copolymeren in kosmetischen Zubereitungen und die entsprechenden kosmetischen Zubereitungen.

## Beschreibung

Die vorliegende Erfindung betrifft die Verwendung von Imidazoliumalkyl(meth)acrylat-Homo- und Copolymeren in kosmetischen Zubereitungen und die entsprechenden kosmetischen Zubereitungen.

N-Vinylimidazol-Polymere werden für unterschiedlichste Zwecke in kosmetischen Zubereitungen, als Farbübertragungsinhibitoren, zur Behandlung von Oberflächen und in der Elektronikindustrie eingesetzt. Der Imidazolrest übernimmt dabei zahlreiche Funktionen wie beispielsweise Komplexierung und Löslichkeitserhöhung.
Schwierigkeiten bei der Herstellung der N-Vinylimidazol-Polymere bereitet mitunter die geringe Neigung des N-Vinylimidazols zur Copolymerisation mit anderen Monomeren.

WO 2006/026064 beschreibt Polymere, die ausgehend von ionischen Flüssigkeiten hergestellt werden. Zweck ist es, eine kontrollierte Adsorption und Desorption von CO₂, das eine hohe Affinität zu Polymeren von ionischen Flüssigkeiten zeigt, zu ermöglichen. Beschrieben werden für diesen Zweck Polymere auf Basis von 1-[2-(methacryloyloxy)ethyl]-3-butyl-imidazolium-Tetrafluoroborat.

Wagner et al. (Research Disclosure Nr. 24315, Juli 1984) beschreiben die Herstellung von 2-(1-Imidazolyl)ethylmethacrylat (im Folgenden als ImEMA bezeichnet), dessen Homopolymer und die Copolymerisation mit Methylmethacrylat sowie die Quaternisierung der Polymere. Beschrieben wird die Verwendung in Beizen für die Fotographie.

Simmons et al. (Macromolecules 1998, 31, 9075-9077) beschreiben die Herstellung von ImEMA, dessen Homopolymerisation und Copolymerisation mit Dimethylaminoethylmethacrylat (DMAEMA) zu Diblock- und statistischen Copolymeren. Beschrieben wird die enzymanaloge Verwendung dieser Polymere zur Hydrolyse.

Simmons et al. (J. Polym. Sci. A 1999, 37, 1501-1512) beschreiben die Herstellung von ImEMA und dessen Oligomerisierung bzw. Homopolymerisation. Untersucht wird die hydrolytische Katalyse-Aktivität dieser Verbindungen.

Patrickios et al. (Colloids & Surfaces A 2000, 167, 61-72) beschreiben lineare und vernetzte ImEMA-Homo- und Copolymere mit DMAEMA oder Methylmethacrylat (MMA). Untersucht wird auch hier die hydrolytische Katalyse-Aktivität dieser Verbindungen.

Hadjikallis et al. (Polymer 2002, 43, 7269-7273) beschreiben die Herstellung von ImEMA-tetrahydropyranylmethacrylat-Blockcopolymeren und deren Hydrolyse zu amphiphilen ImEMA- Methacrylsäure -Blockcopolymeren.

Eine Aufgabe der vorliegenden Erfindung war es, kationische oder kationogene Polymere bereitzustellen, die sowohl mit hohen als auch mit niedrigen Molekulargewichten auf einfachem Weg herstellbar sind und vorteilhafte Eigenschaften bei ihrer Verwendung in der Kosmetik besitzen.

Gelöst wurde diese Aufgabe durch die Verwendung von Polymeren, die wenigstens eine Verbindung der allgemeinen Formel (I) und/oder ihrer kationischen Form (la) einpolymerisiert enthalten 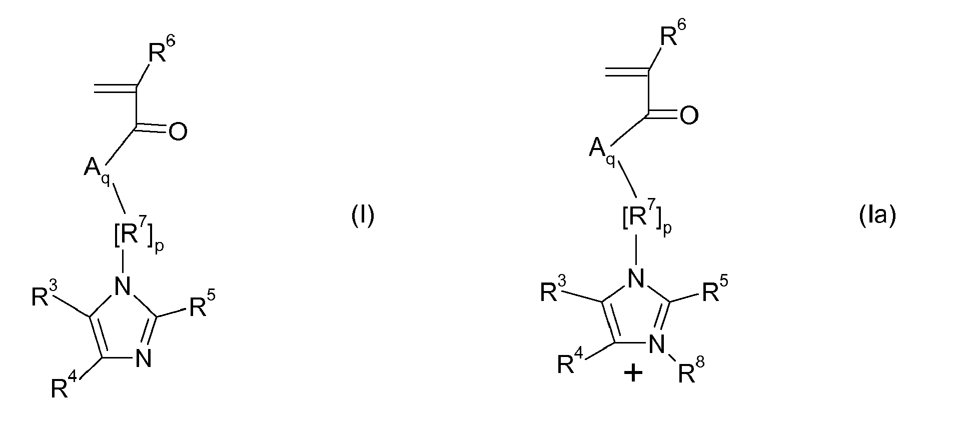 wobei
R³, R⁴ und R⁵ unabhängig voneinander H oder C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₃-C₁₅-Cycloalkylcarbonyl, Aryl, Arylcarbonyl, ein Heterocyclus oder ein Halogenatom
R⁶ H oder Methyl
R⁷ zweiwertiger organischer Rest wie beispielsweise Alkylen oder Arylen
R⁸ H oder C₁-C₁₀-Alkyl
A O oder NH
p, q unabhängig voneinander 0 oder 1 bedeuten,

in kosmetischen Zubereitungen. Insbesondere handelt es sich bei den kosmetischen Zubereitungen um Zubereitungen für die Haarpflege.

In einer bevorzugten Ausführungsform der Erfindung bedeuten p und q nicht gleichzeitig 0.

Bevorzugt handelt es sich bei den Verbindungen (I) bzw. (la) um (Meth)acrylsäureester von Verbindungen der folgenden allgemeinen Formel (II). 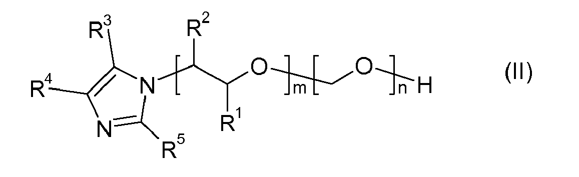 worin
R¹ und R² unabhängig voneinander Wasserstoff oder C₁-C₂₀-Alkyl sein können,
R³, R⁴ und R⁵ unabhängig voneinander Wasserstoff oder C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₃-C₁₅-Cycloalkylcarbonyl, Aryl, Arylcarbonyl, ein Heterocyclus oder ein Halogenatom sein können, und
m und n jeweils ganze Zahlen im Bereich von 0 bis 20 sind, wobei m und n nicht gleichzeitig 0 sein können,
und worin die jeweiligen Einheiten, die von den Variablen m und n umklammert sind, in beliebiger Reihenfolge enthalten sind,
und worin im Falle m ≥ 2 die Reste R¹ und R² jeweils in den jeweiligen Einheiten unabhängig voneinander sind.

Der Begriff "erfindungsgemäß zu verwendende Polymere" steht im Folgenden für alle Polymere, die Verbindungen der Formel (I) und/oder (la) einpolymerisiert enthalten. Dabei kann es sich um statistische, Gradienten-, Block- oder Pfropfcopolymere handeln, die linear oder verzweigt sein können.

Im einzelnen haben die verschiedenen Reste R angegebenen Sammelbegriffe folgende Bedeutung:

C₁-C₂₀-Alkyl: geradkettige oder verzweigte Kohlenwasserstoffreste mit bis zu 20 Kohlenstoffatomen, bevorzugt C₁-C₁₀-Alkyl wie Methyl, Ethyl, Propyl, Isopropyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, 2-Ethylhexyl, 2,4,4-Trimethylpentyl, 1,1,3,3-Tetramethylbutyl, Nonyl und Decyl sowie deren Isomere.

C₁-C₂₀-Alkylcarbonyl: eine geradkettige oder verzweigte Alkylgruppe mit 1 bis 20 Kohlenstoffatomen (wie vorstehend genannt), welche über eine Carbonylgruppe (-CO-) an gebunden ist, bevorzugt C₁-C₁₀-Alkylcarbonyl wie beispielsweise Formyl, Acetyl, n-oder iso-Propionyl, n-, iso-, sec- oder tert.-Butanoyl, n-iso-, sec- oder tert.-Pentanoyl, n- oder iso-Nonanoyl, n-Dodecanoyl.

C₂-C₂₀-Alkenyl: ungesättigte, geradkettige oder verzweigte Kohlenwasserstoffreste mit 2 bis 20 Kohlenstoffatomen und einer Doppelbindung in einer beliebigen Position, bevorzugt C₂-C₁₀-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, sowie die Isomere von Heptenyl, Octenyl, Nonenyl und Decenyl.

C₂-C₂₀-Alkenylcarbonyl: ungesättigte, geradkettige oder verzweigte Kohlenwasserstoffreste mit 2 bis 20 Kohlenstoffatomen und einer Doppelbindung in einer beliebigen Position (wie vorstehend genannt), welche über eine Carbonylgruppe (-CO-) an gebunden sind, bevorzugt C₂-C₁₀-Alkylcarbonyl wie beispielsweise Ethenoyl, Propenoyl, Butenoyl, Pentenoyl, Nonenoyl sowie deren Isomere.

C₂-C₂₀-Alkinyl: geradkettige oder verzweigte Kohlenwasserstoffgruppen mit 2 bis 20 Kohlenstoffatomen und einer Dreifachbindung in einer beliebigen Position, bevorzugt C₂-C₁₀-Alkinyl wie Ethinyl 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl sowie die Isomere von Heptinyl, Octinyl, Noninyl, Decinyl.

C₂-C₂₀-Alkinylcarbonyl: ungesättigte, geradkettige oder verzweigte Kohlenwasserstoffreste mit 2 bis 20 Kohlenstoffatomen und einer Dreifachbindung in einer beliebigen Position (wie vorstehend genannt), welche über eine Carbonylgruppe (-CO-) an gebunden sind, bevorzugt C₂-C₁₀-Alkinylcarbonyl wie beispielsweise Propinoyl, Butinoyl, Pentinoyl, Noninoyl, Decinoyl sowie deren Isomere.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl und Cyclooctyl sowie ein gesättigtes oder ungesättigte cyclisches System wie z. B. Norbornyl oder Norbenyl.

C₃-C₁₅-Cycloalkylcarbonyl: monocyclische, gesättigte Kohlenwasserstoffgruppen mit 3 bis 15 Kohlenstoffringgliedern (wie vorstehend genannt), welche über eine Carbonylgruppe (-CO-) an sind, bevorzugt C₃-C₈-Cycloalkylcarbonyl.

Aryl: ein ein- bis dreikerniges aromatisches Ringsystem enthaltend 6 bis 14 Kohlenstoffringglieder, z. B. Phenyl, Naphthyl und Anthracenyl, bevorzugt ein ein- bis zweikerniges, besonders bevorzugt ein einkerniges aromatisches Ringsystem.

Arylcarbonyl: bevorzugt ein ein- bis dreikerniges aromatisches Ringsystem (wie vorstehend genannt), welches über eine Carbonylgruppe (-CO-) gebunden ist, wie z. B. Benzoyl, bevorzugt ein ein- bis zweikerniges, besonders bevorzugt ein einkerniges arormatisches Ringsystem.

Heterocyclen: fünf- bis zwölfgliedrige, bevorzugt fünf- bis neungliedrige, besonders bevorzugt fünf- bis sechsgliedrige Sauerstoff-, Stickstoff- und/oder Schwefelatome aufweisender gegebenenfalls mehrere Ringe aufweisendes Ringsystem wie beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl.

Die im einzelnen aufgeführten Substituenten können jeweils an beliebiger Position durch ein oder mehrere Heteroatome unterbrochen sein, wobei die Anzahl dieser Heteroatome nicht mehr als 10, bevorzugt nicht mehr als 8, ganz besonders bevorzugt nicht mehr als 5 und insbesondere nicht mehr als 3 beträgt, und/oder jeweils an beliebiger Position, allerdings nicht mehr als fünfmal, bevorzugt nicht mehr als viermal und besonders bevorzugt nicht mehr als dreimal, durch Alkyl, Alkyloxy, Alkyloxycarbonyl, Aryl, Aryloxy, Aryloxycarbonyl, Hydroxycarbonyl, Aminocarbonyl, Heterocyclen, Heteroatome oder Halogenatome substituiert sein können, wobei diese ebenfalls maximal zweimal, bevorzugt maximal einmal mit den genannten Gruppen substituiert sein können.

Die in dieser Gruppe genannten Verbindungsklassen Alkyl, Aryl und Heterocyclen haben die zuvor genannte Bedeutung.

Heteroatome sind Sauerstoff, Stickstoff, Schwefel oder Phosphor.

Alkyloxy bedeutet eine geradkettige oder verzweigte Alkylgruppe mit 1 bis 20 Kohlenstoffatomen (wie vorstehend genannt), welche über ein Sauerstoffatom (-O-) an gebunden sind, bevorzugt C₁-C₁₀-Alkyloxy wie beispislweise Methoxy, Ethoxy, Propoxy.

Alkoxycarbonyl ist eine Alkoxygruppe mit 1 bis 20 Kohlenstoffatomen (wie vorstehend genannt), welche über eine Carbonylgruppe (-CO-) an gebunden ist, bevorzugt C₁-C₁₀-Alkyloxycarbonyl.

Aryloxy ist ein ein- bis dreikerniges aromatisches Ringsystem (wie vorstehend genannt), welches über ein Sauerstoffatom (-O-) an gebunden ist, bevorzugt ein ein- bis zweikerniges, besonders bevorzugt ein einkerniges aromatisches Ringsystem.

Aryloxycarbonyl ist eine ein- bis dreikernige Aryloxygruppe (wie vorstehend genannt), welche über eine Carbonylgruppe (-CO-) an gebunden ist, bevorzugt ein ein- bsi zweikerniges, besonders bevorzugt ein einkerniges Aryloxycarbonyl.

Halogenatome sind Fluor, Chlor, Brom und Iod.

Im Falle der genannten aliphatischen Substituenten können die Reste R³ und R⁴ auch miteinander verbunden sein und so gemeinsam einen drei- bis achtgliedrigen, bevorzugt einen fünf- bis siebengliedrigen und besonders bevorzugt einen fünf- bis sechsgliedrigen Ring bilden.

Weiterhin können die Substituenten jeweils an beliebiger Position durch ein oder mehrere Heteroatome unterbrochen sein können, wobei die Anzahl dieser Heteroatome nicht mehr als 10 beträgt, bevorzugt nicht mehr als 8, besonders bevorzugt nicht mehr als 5 und insbesondere nicht mehr als 3. Heteroatome sind Sauerstoff, Stickstoff, Schwefel oder Phosphor.

In einer bevorzugte Ausführungsform sind die Reste R³, R⁴ und R⁵ jeweils unabhängig voneinander Wasserstoff oder C₁-C₁₀-Alkyl, bevorzugt Wasserstoff oder C₁-C₆-Alkyl, und insbesondere bevorzugt Wasserstoff oder C₁-C₄-Alkyl. Ganz besonders bevorzugt sind R³, R⁴ und R⁵ Wasserstoff, Methyl oder Ethyl.

In einer besonders bevorzugten Ausführungsform sind die Reste R³, R⁴ und R⁵ gleich und sind Wasserstoff, Methyl oder Ethyl.

In einer bevorzugten Ausführungsform sind die Reste R¹ und R² jeweils unabhängig voneinander Wasserstoff oder C₁-C₁₀-Alkyl, bevorzugt Wasserstoff oder C₁-C₆-Alkyl, und insbesondere bevorzugt Wasserstoff oder C₁-C₄-Alkyl. Ganz besonders bevorzugt sind R¹ und R² unabhängig voneinander Wasserstoff oder Methyl.

Die jeweiligen Einheiten, die von den Variablen m und n umklammert sind, können in beliebiger Reihenfolge enthalten sein.

Im Falle von m ≥ 2 können die jeweiligen Einheiten, die von der Variablen m umklammert sind, jeweils unterschiedliche oder gleiche Reste R¹ und R² tragen, so dass solche N-hydroxyalkylierten Imidazole (I) in beliebiger Reihenfolge Ethylen- und Propylenoxid-Einheiten enthalten.

In einer anderen bevorzugten Ausführungsform sind m und n jeweils eine ganze Zahl von 0 bis 10, besonders bevorzugt 0 bis 8 und insbesondere bevorzugt 0 bis 4, wobei wie oben erwähnt M und n nicht gleichzeitig 0 sein können.

Geeignete Monomere sind die (Meth)acrylsäureester von N-hydroxyalkylierten einfach alkylierten Imidazolen wie beispielsweise N-Hydroxymethyl-Imidazol und N-Hydroxyethyl-Imidazol. Weitere geeignete Monomere sind die (Meth)acrylsäureester von Imidazolen, die zwei oder mehr Alkoxyeinheiten, bevorzugt genau zwei Alkoxyeinheiten enthalten.

Verschiedene Wege zur Herstellung der vorgenannten Verbindungen (I), beispielsweise durch Veresterung von Verbindungen (II) mit (Meth)acrylsäure, sind in der Europäischen Patentanmeldung Nr. 07102757.7 beschrieben, worauf hiermit in vollem Umfang Bezug genommen wird.

Bevorzugte Polymere enthalten Verbindungen der Formel (I) einpolymerisiert, für die folgende Bedingungen gelten:

| R³ | R⁴ | R⁵ | R⁶ | R⁷ | A | q | p |
|---|---|---|---|---|---|---|---|
| H | H | H | CH₃ | CH₂ | O | 1 | 1 |
| H | H | H | CH₃ | C₂H₄ | O | 1 | 1 |
| H | H | H | CH₃ | CH₂ | NH | 1 | 1 |
| H | H | H | CH₃ | C₂H₄ | NH | 1 | 1 |
| H | H | H | CH₃ | - | - | 0 | 0 |
| | | | | | | | |
| H | H | H | H | CH₂ | O | 1 | 1 |
| H | H | H | H | C₂H₄ | O | 1 | 1 |
| H | H | H | H | CH₂ | NH | 1 | 1 |
| H | H | H | H | C₂H₄ | NH | 1 | 1 |
| H | H | H | H | - | - | 0 | 0 |

Weiter bevorzugte Polymere enthalten (Meth)acrylsäureester von N-hydroxyalkylierten Imidazolen einpolymerisiert.

Besonders bevorzugte Polymere enthalten (2-(1-Imidazolyl)ethylmethacrylat) einpolymerisiert (R⁶=CH₃, R³ bis R⁵ = H, p=q=1, A=O und R⁷=C₂H₄). Die Herstellung von 2-(1-Imidazolyl)ethylmethacrylat ist dem Fachmann bekannt und beispielsweise in der Europäischen Patentanmeldung Nr. 07102757.7 beschrieben.

Die Polymere können die Verbindungen der Formel (I) auch in der jeweils kationischen Form der Formel (la) einpolymerisiert enthalten. Dabei bedeutet R⁸ bevorzugt CH₃ oder C₂H₅. Die Verbindungen der Formel (la) lassen sich durch Quaternisierung mit Alkylierungsmitteln erzeugen. Geeignete Alkylierungsmittel sind beispielsweise C₅-C₃₀-Alkylhalogenide, insbesondere -Bromide und -Chloride, bevorzugt sind C₁-C₄-Alkylhalogenide oder -sulfate, wie Ethylchlorid, Ethylbromid, Methylchlorid, Methylbromid, Dimethylsulfat und Diethylsulfat. Ein bevorzugtes Quaternisierungsmittel ist Methylchlorid. Ein weiteres bevorzugtes Quaternisierungsmittel ist Diethylsulfat.

Geladene kationische Gruppen lassen sich auch durch Protonierung der Aminstickstoffatome mit Säuren erzeugen. Geeignete Säuren sind z. B. Carbonsäuren, beispielsweise Milchsäure, oder Mineralsäuren, beispielsweise Phosphorsäure, Schwefelsäure und Salzsäure.
Selbstverständlich wird die kationische Ladung von Verbindungen der Formel (la) regelmäßig durch Anionen neutralisiert. Diese Anionen sind bevorzugt Chlorid, Bromid, Methylsulfat (CH₃-O-SO₃⁻ - Anion), Ethylsulfat (C₂H₅-O-SO₃⁻ - Anion) und Sulfat (-SO₄²⁻).

Die zuvor beschriebenen Polymere können auch nach ihrer Herstellung durch teilweise oder vollständige Quaternisierung und/oder Protonierung in die kationische Form überführt werden. Generell sind dafür die gleichen Quaternisierungsmittel geeignet wie für die Quaternisierung der monomeren Verbindungen.

Die einpolymerisierten Verbindungen der Formel (I) können im Polymer teilweise oder vollständig quaternisiert sein.

In einer bevorzugten Ausführungsform der Erfindung werden Homopolymere der Verbindungen (I) bzw. (la) in kosmetischen Zubereitungen verwendet.

In einer bevorzugten Ausführungsform der Erfindung werden Copolymere der Verbindungen (I) bzw. (la) verwendet. Diese Copolymere sind erhältlich durch die Polymerisation von Monomermischungen, die neben den Verbindungen der Formel (I) bzw. (la) weiteren radikalisch polymerisierbare Verbindungen umfassen.
Prinzipiell kommen dafür alle radikalisch polymerisierbare Verbindungen in Betracht insofern sich kosmetisch akzeptable Polymere synthetisieren lassen.

Bevorzugte weitere radikalisch polymerisierbare Verbindungen sind beispielsweise offenkettige N-Vinylamidverbindungen wie N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon.
Weitere bevorzugte radikalisch polymerisierbare Verbindungen sind ausgewählt aus der Gruppe bestehend aus Acrylamid, den N-Vinylderivaten von gegebenfalls alkylsubstituiertem 2-Pyrrolidon, gegebenfalls alkylsubstituiertem 2-Piperidon und gegebenfalls alkylsubstituiertem ε-Caprolactam.
Weiterhin bevorzugte radikalisch polymerisierbare Verbindungen sind ausgewählt aus der Gruppe bestehend aus den N-Vinylderivaten von 2-Pyrrolidon, 3-Methyl-2-pyrrolidon, 4-Methyl-2-pyrrolidon, 5-Methyl-2-pyrrolidon, 3-Ethyl-2-pyrrolidon, 3-Propyl-2-pyrrolidon, 3-Butyl-2-pyrrolidon, 3,3-Dimethyl-2-pyrrolidon, 3,5-Dimethyl-2-pyrrolidon, 5,5-Dimethyl-2-pyrrolidon, 3,3,5-Trimethyl-2-pyrrolidon, 5-Methyl-5-ethyl-2-pyrrolidon, 3,4,5-Trimethyl-2-pyrrolidon, 3-Methyl-2-piperidon, 4-Methyl-2-piperidon, 5-Methyl-2-piperidon, 6-Methyl-2-piperidon, 6-Ethyl-2-piperidon, 3,5-Dimethyl-2-piperidon, 4,4-Dimethyl-2-piperidon, 3-Methyl-ε-caprolactam, 4-Methyl-ε-caprolactam, 5-Methyl-εcaprolactam, 6-Methyl-ε-caprolactam, 7-Methyl-ε-caprolactam, 3-Ethyl-ε-caprolactam, 3-Propyl-ε-caprolactam, 3-Butyl-ε-caprolactam, 3,3-Dimethyl-ε-caprolactam, 7,7-Dimethyl-ε-caprolactam und deren Mischungen.
Besonders bevorzugt als wenigstens eine weitere radikalisch polymerisierbare Verbindung ist N-Vinylpyrrolidon, Methyl-N-Vinylpyrrolidon oder N-Vinylcaprolactam.

Bevorzugte weitere radikalisch polymerisierbare Verbindungen sind am Stickstoff ein-oder zweifach C₁-C₂₄-Alkyl substituierte Ester der (Meth)acrylsäure mit Aminoalkoholen. Besonders bevorzugt sind diese ausgewählt aus der Gruppe bestehend aus N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Besonders bevorzugt ist N,N-Dimethylaminoethylmethacrylat.

Weitere bevorzugte radikalisch polymerisierbare Verbindungen sind am Stickstoff ein-oder zweifach C₁-C₂₄-Alkyl substituierte Amide der (Meth)acrylsäure mit Diaminen. Besonders bevorzugt sind diese ausgewählt aus der Gruppe bestehend aus N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)- butyl]methacrylamid, N-[2-(diethylamino)ethyl]acrylamid, N-[4-(dimethylamino)cyclohexyl]acrylamid, N-[4-(dimethylamino)cyclohexyl]methacrylamid, N-[8-(Dimethylamino)octyl]methacrylamid, N-[12-(Dimethylamino)dodecyl]methacrylamid, N-[3-(Diethylamino)propyl]methacrylamid und N-[3-(Diethylamino)propyl]acrylamid. Besonders bevorzugt ist N-[3-(dimethylamino)propyl]methacrylamid.

Geeignete radikalisch polymerisierbare Verbindungen sind weiterhin N,N-Diallylamine und N,N-Diallyl-N-alkylamine und deren Säureadditionssalze und Quaternisierungsprodukte. Alkyl steht dabei vorzugsweise für C₁-C₂₄-Alkyl. Bevorzugt sind N,N-Diallyl-N-methylamin und N,N-Diallyl-N,N-dimethylammonium-Verbindungen, wie z. B. die Chloride und Bromide. Dazu zählt insbesondere N,N-Diallyl-N-methylamin sowie dessen methyliertes Derivat N,N-Diallyl-N,N-Dimethylammoniumchlorid (DADMAC).

Geeignete Monomere sind weiterhin vinyl- und allylsubstituierte Stickstoffheterocyclen, wie 2- und 4-Vinylpyridin, 2- und 4-Allylpyridin, und die Salze davon.

Weiterhin können als radikalisch polymerisierbare Verbindungen α,β-ethylenisch ungesättigte Monomere der allgemeinen Formel (III) einpolymerisiert werden 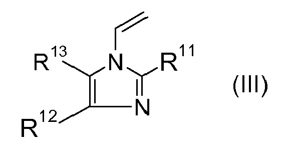 wobei R¹¹ bis R¹³ unabhängig voneinander Wasserstoff, C₁-C₄-Alkyl oder Phenyl bedeuten.

Beispiele für geeignete Verbindungen der allgemeinen Formel (III) sind folgender Tabelle 1 zu entnehmen:

**Tabelle 1**

| R¹¹ | R¹² | R¹³ |
|---|---|---|
| | | |
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt sind 1-Vinylimidazol (N-Vinylimidazol) und Mischungen, die N-Vinylimidazol enthalten.

Geeignete radikalisch polymerisierbare Verbindungen sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Geeignete radikalisch polymerisierbare Verbindungen sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Geeignete radikalisch polymerisierbare Verbindungen sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20000 auf. Geeignete Polyetherole sind Polyalkylenglykole, wie Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.

Geeignete radikalisch polymerisierbare Verbindungen sind auch Polyetheracrylate der allgemeinen Formel IV 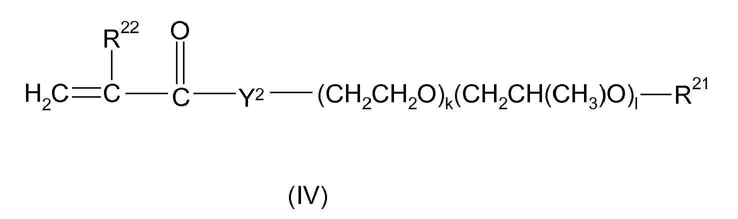 worin
die Reihenfolge der Alkylenoxideinheiten beliebig ist,
k und I unabhängig voneinander für eine ganze Zahl von 0 bis 1000 stehen, wobei die Summe aus k und I mindestens 5 beträgt,
R²¹ für Wasserstoff, C₁-C₃₀-Alkyl, C₅-C₈-Cycloalkyl oder Aryl steht,
R²² für Wasserstoff oder C₁-C₈-Alkyl steht,
Y² für O oder NR²³ steht, wobei R²³ für Wasserstoff, C₁-C₃₀-Alkyl oder C₅-C₈-Cycloalkyl steht. Bevorzugt steht k für eine ganze Zahl von 1 bis 500, insbesondere 3 bis 250. Bevorzugt steht I für eine ganze Zahl von 0 bis 100. Bevorzugt steht R²² für Wasserstoff, Methyl, Ethyl, n-Propyl, Isopropyl, n-Butyl, sek.-Butyl, tert.-Butyl, n-Pentyl oder n-Hexyl, insbesondere für Wasserstoff, Methyl oder Ethyl. Vorzugsweise steht R²¹ in der Formel IV für Wasserstoff, Methyl, Ethyl, n-Propyl, Isopropyl, n-Butyl, sec-Butyl, n-Pentyl, n-Hexyl, Octyl, 2-Ethylhexyl, Decyl, Lauryl, Palmityl oder Stearyl, Phenyl. Vorzugsweise steht Y² in der Formel IV für O oder NH.

Geeignete Polyetheracrylate der vorgenannten Art sind z. B. die Polykondensationsprodukte der zuvor genannten α,β-ethylenisch ungesättigten Mono- und/oder Dicarbonsäuren und deren Säurechloride, -amide und Anhydride mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R²¹-OH hergestellt werden. Die Alkylenoxide können einzeln, alternierend nacheinander oder als Mischung eingesetzt werden. Die Polyetheracrylate können allein oder in Mischungen zur Herstellung der erfindungsgemäß verwendeten Polymere verwendet werden.

Geeignete zusätzliche radikalisch polymerisierbare Verbindungen sind Methyl(meth)acrylat, Methylethacrylat, Ethyl(meth)acrylat, Ethylethacrylat, n-Propyl(meth)acrylat, i-Propyl(meth)acrylat, n-Butyl(meth)acrylat, tert.-Butylmethacrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat,Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.
Bevorzugte radikalisch polymerisierbare Verbindungen sind die Ester α,β-ethylenisch ungesättigter Mono- und Dicarbonsäuren mit C₁-C₄-Alkanolen.

Geeignete zusätzliche radikalisch copolymerisierbare Verbindungen sind weiterhin Vinylacetat, Vinylpropionat, Vinylbutyrat und Mischungen davon.

Geeignete zusätzliche radikalisch copolymerisierbare Verbindungen sind weiterhin Methylvinylether, Ethylvinylether, n-Propylvinylether, Isopropylvinylether, n-Butylvinylether, Isobutylvinylether, 2-Ethylhexylvinylether, Dodecylvinylether und Octadecylvinylether.

Geeignete zusätzliche radikalisch copolymerisierbare Verbindungen sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Bevorzugte radikalisch copolymerisierbare Verbindungen sind Verbindungen mit einer radikalisch polymerisierbaren, α,β-ethylenisch ungesättigten Doppelbindung und wenigstens einer anionogenen und/oder anionischen Gruppe pro Molekül.

Bevorzugte radikalisch copolymerisierbare Verbindungen sind Verbindungen, die ausgewählt sind unter monoethylenisch ungesättigten Carbonsäuren, Sulfonsäuren, Phosphonsäuren und Mischungen davon. Dazu zählen monoethylenisch ungesättigte Mono- und Dicarbonsäuren mit 3 bis 25 vorzugsweise 3 bis 6 C-Atomen, die auch in Form ihrer Salze oder Anhydride eingesetzt werden können. Beispiele hierfür sind Acrylsäure, Methacrylsäure, Methacrylsäureanhydrid, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Verbindungen mit einer radikalisch polymerisierbaren, α,β-ethylenisch ungesättigten Doppelbindung und wenigstens einer anionogenen und/oder anionischen Gruppe pro Molekül zählen weiterhin die Halbester von monoethylenisch ungesättigten Dicarbonsäuren mit 4 bis 10 vorzugsweise 4 bis 6 C-Atomen, z. B. von Maleinsäure wie Maleinsäuremonomethylester. Zu den Verbindungen mit einer radikalisch polymerisierbaren, α,β-ethylenisch ungesättigten Doppelbindung und wenigstens einer anionogenen und/oder anionischen Gruppe pro Molekül zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Weiterhin zählen dazu auch die Salze der zuvor genannten Säuren, insbesondere die Natrium-, Kalium- und Ammoniumsalze sowie die Salze mit Aminen. Diese Monomere können als solche oder als Mischungen eingesetzt werden.
Wird eine derartige anionische oder anionogene Verbindung zur Copolymerisation ausgewählt, so wird sie bevorzugt ausgewählt aus der Gruppe bestehend aus Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure, 2-Acrylamido-2-methylpropansulfonsäure, Vinylphosphonsäure und Mischungen davon. Besonders bevorzugt ist die zur Copolymerisation eingesetzte anionische oder anionogene Verbindung ausgewählt unter Acrylsäure, Methacrylsäure und Mischungen davon.

Eine bevorzugte Ausführungsform der Erfindung ist die erfindungsgemäße Verwendung der zuvor beschriebenen Polymere, die wenigstens eine anionische oder anionogene Verbindung einpolymerisiert enthalten.

Eine weitere bevorzugte Ausführungsform der Erfindung ist die erfindungsgemäße Verwendung der zuvor beschriebenen Polymere, die wenigstens eine kationische oder kationogene Verbindung einpolymerisiert enthalten.

Eine weitere bevorzugte Ausführungsform der Erfindung ist die erfindungsgemäße Verwendung der zuvor beschriebenen Polymere, die wenigstens eine nichtionische Verbindung einpolymerisiert enthalten.

Eine weitere bevorzugte Ausführungsform der Erfindung ist die erfindungsgemäße Verwendung der zuvor beschriebenen Polymere, die wenigstens eine anionische oder anionogene und wenigstens eine kationische oder kationogene Verbindung einpolymerisiert enthalten.

Eine weitere bevorzugte Ausführungsform der Erfindung ist die erfindungsgemäße Verwendung der zuvor beschriebenen Polymere, die wenigstens eine anionische oder anionogene und wenigstens eine nichtionische Verbindung einpolymerisiert enthalten.

Eine weitere bevorzugte Ausführungsform der Erfindung ist die erfindungsgemäße Verwendung der zuvor beschriebenen Polymere, die wenigstens eine kationische oder kationogene und wenigstens eine nichtionische Verbindung einpolymerisiert enthalten.

Eine weitere bevorzugte Ausführungsform der Erfindung ist die erfindungsgemäße Verwendung der zuvor beschriebenen Polymere, die wenigstens eine anionische oder anionogene und wenigstens eine kationische oder kationogene und wenigstens eine nichtionische Verbindung einpolymerisiert enthalten.

Bevorzugt ist die erfindungsgemäße Verwendung der Polymere, wobei das wenigstens eine weitere Monomer ausgewählt ist aus N-Vinyllactamen, Säuregruppen enthaltenden Monomeren, (Meth)acrylsäureestern, (Meth)acrylsäureamiden, Vinylethern und Diallylaminen.

Besonders bevorzugt ist die erfindungsgemäße Verwendung der Polymere, wobei das wenigstens eine weitere Monomer ausgewählt ist aus N-Vinylpyrrolidon, Acrylsäure, Methylmethacrylat, N,N-Diallyl-N,N-Dimethylammoniumchlorid, N,N-Dimethylaminoethylmethacrylat, N-[3-(dimethylamino)propyl]methacrylamid, Methacrylamid und deren Mischungen.

Bevorzugt ist die erfindungsgemäße Verwendung, wobei die Polymere wenigstens zwei weitere Monomere einpolymerisiert enthalten, wobei wenigstens eines der weiteren Monomere ausgewählt ist aus N-Vinyllactamen und wenigstens eines der weiteren Monomere ausgewählt ist aus (Meth)acrylsäureamiden.

Insbesondere bevorzugt ist die erfindungsgemäße Verwendung von Polymeren analog Tabelle 2, S.43 der WO 03/092640, wobei das dort einpolymerisierte Vinylimidazol erfindungsgemäß teilweise oder vollständig durch ImEMA ersetzt wird. Auf die Offenbarung der der WO 03/092640 wird hiermit in vollem Umfang Bezug genommen.

### Vernetzer

Die erfindungsgemäß verwendeten Copolymere können gewünschtenfalls wenigstens einen Vernetzer, d. h. eine Verbindung mit zwei oder mehr als zwei ethylenisch ungesättigten, nichtkonjugierten Doppelbindungen einpolymerisiert enthalten.

Vorzugsweise werden Vernetzer in einer Menge von 0,01 bis 2 Gew.-%, besonders bevorzugt 0,1 bis 1 Gew.-%, bezogen auf das Gesamtgewicht der zur Polymerisation eingesetzten Monomere, verwendet.

Geeignete Vernetzer sind zum Beispiel Acrylester, Methacrylester, Allylether oder Vinylether von mindestens zweiwertigen Alkoholen. Die OH-Gruppen der zugrunde liegenden Alkohole können dabei ganz oder teilweise verethert oder verestert sein; die Vernetzer enthalten aber mindestens zwei ethylenisch ungesättigte Gruppen.

Beispiele für die zugrunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrunde liegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Weitere geeignete Vernetzer sind Urethandiacrylate und Urethanpolyacrylate, wie sie z. B. unter der Bezeichnung Laromer® kommerziell erhältlich sind.

Geeignet als Vernetzer sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer sind Divinyldioxan, Tetraallylsilan oder Tetravinylsilan.

Selbstverständlich können auch Mischungen der vorgenannten Vernetzer eingesetzt werden.
Besonders bevorzugt eingesetzte Vernetzer sind beispielsweise Methylenbisacrylamid, Triallylamin und Triallylalkylammoniumsalze, Divinylimidazol, Pentaerythrittriallylether, N,N'-Divinylethylenharnstoff, Trimethylolpropandiallylether, Allylsucrose, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Pentaerythrittriallylether, Trimethylolpropandiallylether, Allylsucrose, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

Die Vernetzung kann bei Vorliegen von quaternisierbaren Gruppen im Polymer auch durch Umsetzung des Polymers mit Alkylierungsmitteln, die zur zweifachen Alkylierung geeignet sind, erfolgen.

Die für die erfindungsgemäße Verwendung geeigneten unvernetzten Polymere besitzen bevorzugt massenmittlere Molekulargewichte M_{w} im Bereich von 10 000 bis 5 Millionen, weiter bevorzugt von 30 000 bis 2 Millionen, besonders bevorzugt von 500 000 bis 1,5 Millionen g/mol.

Die für die erfindungsgemäße Verwendung geeigneten unvernetzten Polymere besitzen bevorzugt zahlenmittlere Molekulargewichte Mₙ im Bereich von 10 000 bis 500 000, weiter bevorzugt von 40 000 bis 250 000 g/mol.

### Lösungspolymerisation

Bevorzugt werden die Polymere durch Lösungspolymerisation in wässriger Lösung hergestellt.
In einer weiteren Ausführungsform der Erfindung werden die Polymere durch Lösungspolymerisation in alkoholischer Lösung hergestellt.
In einer weiteren Ausführungsform der Erfindung umfasst das Lösungsmittel Wasser und Alkohol. Geeignete Alkohole sind beispielsweise Methanol, Ethanol, n-Propanol, Isopropanol, n-Butanol, sek.-Butanol, tert.-Butanol, 3-Methyl-1-butanol (Isoamylalkohol), n-Hexanol, Cyclohexanol oder Glykole wie Ethylenglykol, Propylenglykol und Butylenglykol sowie Alkylether mehrwertiger Alkohole wie Diethylenglykol, Triethylenglykol, Polyethylenglykolen mit zahlenmittleren Molekulargewichten bis etwa 3000, Glycerin. Besonders bevorzugt ist oder umfasst der Alkohol Ethanol und/oder Isopropanol, insbesondere Isopropanol.
In einer Ausführungsform der Erfindung liegt der Alkoholanteil an der gesamten Lösungsmittelmenge im Bereich von 0 bis 80 Gew.-%, bevorzugt von 10 bis 70 Gew.-%, besonders bevorzugt von 15 bis 50 Gew.-%, jeweils bezogen auf die Gesamtmenge des Lösungsmittels.
Ein weiteres übliches Lösungsmittel umfasst im Bereich von 60-80 Gew.-% Alkohol und etwa 40-20 Gew.-% Wasser.

Zusätzlich zu Wasser oder Wasser/Alkohol können in der Polymerisationslösung weitere Lösungsmittel enthalten sein. Prinzipiell kommen alle für die radikalische Polymerisation geeigneten Lösungsmittel in Frage wie beispielsweise Aceton, Acetonitril, Anilin, Anisol, Benzonitril, tert-Butylmethylether (TBME), Gamma-Butyrolacton, Chinolin, Chloroform, Cyclohexan, Diethylether, Dimethylacetamid, Dimethylformamid, Dimethylsulfoxid, Dioxan, Essigsäureethylester, Ethylendichlorid, Ethylenglycoldimethylether, Formamid, Hexan, Methylenchlorid, Methylethylketon, N-Methylformamid, Petrolether/Leichtbenzin, Propylencarbonat (4-Methyl-1,3-dioxol-2-on), Sulfolan, Tetrachlorethen, Tetrachlorkohlenstoff, Tetrahydrofuran, Toluol, 1,1,1-Trichlorethan, Trichlorethen, Triethylenglycoldimethylether (Triglyme) und deren Mischungen.

Unter Polymerisationslösung wird diejenige Substanzmischung verstanden, die nach Zugabe aller Komponenten und Beendigung der Polymerisation und vor dem ersten Aufarbeitungsschritt wie beispielsweise einer Trocknung, einer Neutralisation oder einer Wasserdampfdestillation vorliegt.
Die Menge an Wasser liegt bevorzugt im Bereich von 100 bis 50 Gew.-%, bezogen auf das Lösungsmittel.
Die Menge an Alkohol liegt bevorzugt im Bereich von 0 bis 50 Gew.-%, bezogen auf das Lösungsmittel.

Die Menge an weiterhin in der Polymerisationslösung enthaltenen Substanzen, welche im wesentlichen die Monomere, der Initiator und gegebenenfalls Regler und Vernetzer sind, beträgt bevorzugt wenigstens 5, besonders bevorzugt wenigstens 10 und insbesondere wenigstens 20 und bevorzugt höchstens 55, besonders bevorzugt höchstens 50 und insbesondere höchstens 45 Gew.-% der Polymerisationslösung.
Diese Menge wird auch als Feststoffgehalt der Polymerisationslösung bezeichnet.

Bevorzugt ist ein erfindungsgemäßes Verfahren bei dem die Temperatur der Polymerisationslösung im Bereich von 30°C bis 120°C, besonders bevorzugt im Bereich von 50°C bis 110°C und insbesondere im Bereich von 60°C bis 90°C liegt.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 10 bar.

### Fällungspolymerisation

Die erfindungsgemäß zu verwendenden Polymere können auch durch radikalische Fällungspolymerisation hergestellt werden. Die Methode der Fällungspolymerisation an sich ist dem Fachmann bekannt. Bei der Fällungspolymerisation sind die eingesetzten Monomere im Reaktionsmedium (Monomer, Lösungsmittel) löslich, das entsprechende Polymer aber nicht. Das entstehende Polymer wird unter den gewählten Polymerisationsbedingungen unlöslich und fällt aus dem Reaktionsgemisch aus. Die Fällungspolymerisation erfolgt bevorzugt in einem Ester, wie Essigsäureethylester oder Essigsäurebutylester als Lösungsmittel. Die resultierenden Polymerteilchen fallen aus der Reaktionslösung aus und können durch übliche Verfahren, wie Filtration mittels Unterdruck, isoliert werden. Zur Fällungspolymerisation können oberflächenaktive, polymere Verbindungen, vorzugsweise auf Polysiloxanbasis, eingesetzt werden. Bei der Fällungspolymerisation werden in der Regel Polymere mit höheren Molekulargewichten als bei der Lösungspolymerisation erhalten.

Als Initiator für die radikalische Polymerisation in Lösung in Wasser wird bevorzugt wenigstens ein wasserlöslicher Polymerisationsinitiator ausgewählt aus der Gruppe bestehend aus Peroxiden, Hydroperoxiden, Peroxodisulfaten, Percarbonaten, Peroxidestern, Azoverbindungen und deren Mischungen verwendet.
Unter einem wasserlöslichen Polymerisationsinitiator wird ein Initiator verstanden, der bei 20°C und 1013 mbar zu wenigstens 1 g, bevorzugt zu wenigstens 10g in 1 Liter Wasser löslich ist.
In einer bevorzugten Ausführungsform der Erfindung wird der wasserlösliche Polymerisationsinitiator ausgewählt aus der Gruppe bestehend aus wasserlöslichen Azoverbindungen, Wasserstoffperoxid, Lithiumperoxodisulfat, Natriumperoxodisulfat, Kaliumperoxodisulfat, Ammoniumperoxodisulfat und deren Mischungen.
Weiterhin bevorzugt wird der wasserlösliche Polymerisationsinitiator ausgewählt aus der Gruppe bestehend aus
2,2'-Azobis(2-methylpropionamidin)dihydrochlorid
2,2'-Azobis[2-(5-methyl-2-imidazolin-2-yl)propan]dihydrochlorid
2,2'-Azobis[2-(2-imidazolin-2-yl)propan] dihydrochlorid
2,2'-Azobis[2-(2-imidazolin-2-yl)propandisulfatdihydrat
2,2'-Azobis(2-methylpropionamid)dihydrochlorid
2,2'-Azobis[N-(2-carboxyethyl)-2-methylpropionamidin]tetrahydrat
2,2'-Azobis[2-(3,4,5,6-tetrahydropyrimidin-2-yl)propan] dihydrochlorid
2,2'-Azobis{2-[1-(2-hydroxyethyl)-2-imidazolin-2-yl]propan}dihydrochlorid
2,2'-Azobis[2-(2-imidazolin-2-yl)propan]
2,2'-Azobis{2-methyl-N-[1,1-bis(hydroxymethyl)-2-hydroxyethyl]propionamid
2,2'-Azobis{2-methyl-N-[2-(1-hydroxybuthl)]propionamid}
2,2'-Azobis[2-methyl-N-(2-hydroxyethyl)propionamid] und deren Mischungen.

Auch wasserlösliche Redox-Initiatorsysteme können als Polymerisationsinitiatoren verwendet werden. Solche Redox-Initiatorsysteme enthalten mindestens eine peroxidhaltige Verbindung in Kombination mit einem Redox-Coinitiator z.B. reduzierend wirkende Schwefelverbindungen, beispielsweise Bisulfite, Sulfite, Thiosulfate, Dithionite und Tetrathionate von Alkalimetallen und Ammoniumverbindungen. So kann man Kombinationen von Peroxodisulfaten mit Alkalimetall- oder Ammoniumhydrogensulfiten einsetzen, z.B. Ammoniumperoxodisulfat und Ammoniumdisulfit. Die Menge der peroxidhaltigen Verbindung zum Redox-Coinitiator liegt im Bereich von 30:1 bis 0,05:1.

In Kombination mit den Initiatoren bzw. den Redoxinitiatorsystemen können zusätzlich Übergangsmetallkatalysatoren eingesetzt werden, z.B. Salze von Eisen, Kobalt, Nickel, Kupfer, Vanadium und Mangan. Geeignete Salze sind z.B. Eisen (II)sulfat, Kobalt (II)chlorid, Nickel(II)sulfat, oder Kupfer(I)chlorid. Bezogen auf die Monomeren wird das reduzierend wirkende Übergangsmetallsalz in einer Konzentration von 0,1 ppm bis 1000 ppm eingesetzt. So kann man Kombinationen von Wasserstoffperoxid mit Eisen(II)-Salzen einsetzen, wie beispielsweise 0,5 bis 30 % Wasserstoffperoxid und 0,1 bis 500 ppm Mohrsches Salz.

Weiterhin können in Kombination mit den oben genannten Initiatoren Redox-Coinitiatoren und/oder Übergangsmetallkatalysatoren mitverwendet werden, z.B. Benzoin, Dimethylanilin, Ascorbinsäure sowie Komplexe von Schwermetallen, wie Kupfer, Cobalt, Eisen, Mangan, Nickel und Chrom. Die üblicherweise verwendeten Mengen an Redox-Coinitiatoren bzw. Übergangsmetallkatalysatoren betragen etwa 0,1 bis 1000 ppm, bezogen auf die eingesetzten Mengen an Monomeren. Weitere geeignete Initiatoren sind beschrieben in den Kapiteln 20 und 21 von Macromolecules, Vol. 2, 2nd Ed., H. G. Elias, Plenum Press, 1984, New York, worauf hier vollumfänglich Bezug genommen wird. Weiterhin sind geeignete Photoinitiatoren beschrieben in S. P. Pappas, J. Rad. Cur., July 1987, S.6, worauf hier vollumfänglich Bezug genommen wird.

Weitere für die Herstellung der erfindungsgemäß zu verwendenden Polymere geeignete Initiatoren sind ausgewählt aus der Gruppe bestehend aus Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, oder 2-2'-Azo-bis-(2-methyl-butyronitril). Diisobutyrylperoxid, Cumylperoxineodecanoat, Cumylperoxineoheptanoat, tert-Amylperoxyneodecanoat, tert-Butylperoxyneodecanoat, di-sec-Butylperoxidicarbonat, Diisopropylperoxidicarbonat, Diacetylperoxidicarbonat, tert.-Amylperoxiperpivalat, 2,5-Dimethyl-2,5-Di(tert-butylperoxi)hexan, tert.-Butylperoxiacetat, tert.-Butylperoxibenzoat.

Die Menge des wenigstens einen für die Polymerisation der Monomeren eingesetzten wasserlöslichen Initiators beträgt vorzugsweise von 0,0001 bis 10, besonders bevorzugt 0,001 bis 5 und insbesondere 0,02 bis 3 Gew.-%, bezogen auf die Gesamtmenge der eingesetzten Monomere.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, Cystein 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden.

Die Lösungspolymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

In einer Ausführungsform der Erfindung wird die Gesamtmenge der Monomere und gegebenenfalls der Vernetzer und der Regler vorgelegt und der Initiator allmählich zur Reaktionsmischung zugegeben.

In einer weiteren Ausführungsform der Erfindung wird ein Teil eines Monomers, beispielsweise 1 bis 50, bevorzugt 5 bis 40, besonders bevorzugt 10 bis 30 Gew.-% eines Monomers, Lösungsmittel sowie ein Teil des gegebenenfalls verwendeten Reglers, beispielsweise 1 bis 50, bevorzugt 5 bis 40, besonders bevorzugt 10 bis 30 Gew.-% des Reglers vorgelegt und die übrigen Monomere, Lösungsmittel und der restliche Regler in einem Zulauf sowie Lösungsmittel und Initiator in einem zweiten Zulauf allmählich zur Reaktionsmischung zugegeben. Dabei ist es vorteilhaft, sowohl in der Vorlage als auch in den Zuläufen Lösungsmittel zu verwenden.

In einer weiteren Ausführungsform der Erfindung wird der größte Teil eines Monomers, beispielsweise mehr als 50, bevorzugt mehr als 60, weiter bevorzugt mehr als 70, besonders bevorzugt mehr als 80 und insbesondere mehr als 90 Gew.-% vorgelegt und gemeinsam mit Lösungsmittel gegebenenfalls erwärmt. Dann wird ein Teil der übrigen Monomere, beispielsweise bis zu 50, bevorzugt bis zu 40, besonders bevorzugt bis zu 30 und insbesondere bis zu 20 Gew.-% der übrigen Monomere zur gegebenenfalls erwärmten Vorlage gegeben und die Polymerisation mit Hilfe des Initiators gestartet. Die Restmenge der Monomeren und des Initiators wird dann allmählich zudosiert.
In einer bevorzugten Ausführungsform werden die übrigen Monomere als Mischung gemeinsam zudosiert.

Die Polymerisation wird bevorzugt unter weitgehendem Ausschluss von Sauerstoff ausgeführt. Bevorzugt ist es, die Polymerisation unter Schutzgasatmosphäre wie beispielsweise Argon- oder bevorzugt Stickstoffatmosphäre durchzuführen.

Die Polymerisation kann prinzipiell bei dem durch die eingesetzten Monomere resultierenden pH-Wert erfolgen.
Vorzugsweise wird der pH-Wert der Polymerisationslösung auf einen Wert von 5 bis 10, weiter bevorzugt 6 bis 12, besonders bevorzugt 6,5 bis 7,5 eingestellt. Vorzugsweise wird auch der pH-Wert der Vorlage und der verschiedenen Zuläufe auf einen Wert von 5 bis 10, weiter bevorzugt 6 bis 12, besonders bevorzugt 6,5 bis 7,5 eingestellt.
Es ist weiterhin vorteilhaft, den pH-Wert während der Polymerisation dann in diesem Bereich zu halten. Zur Einstellung des pH-Werts vor, während oder nach der Polymerisation eignen sich prinzipiell alle anorganischen oder organischen Basen und Säuren, insbesondere solche, die außer einer etwaigen Salzbildung keine Reaktion mit den Monomeren eingehen. Geeignete Basen sind z. B. Alkali- und Erdalkalihydroxide, Ammoniak, tertiäre Amine, wie Triethylamin, sowie Aminoalkohole, wie Triethanolamin, Methyldiethanolamin oder Dimethylethanolamin. Bevorzugt wird zur Einstellung des pH-Werts NaOH oder wenigstens ein tertiäres Amin, das insbesondere ausgewählt ist unter N,N-Dimethylethanolamin, N-Methyldiethanolamin, Triethanolamin und Mischungen davon, eingesetzt. Geeignete Säuren sind organische Säuren wie Ameisensäure, Essigsäure, Buttersäure, Milchsäure und anorganische Säuren wie beispielsweise Phosphorsäure, Salzsäure, Schwefelsäure oder Salpetersäure.
Jedenfalls ist es vorteilhaft, durch Einstellung eines pH-Wertes im Bereich von 6 bis 8 die Hydrolyse der Monomere möglichst gering zu halten.
Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden, was insbesondere vorteilhaft zur Beseitigung von Komponenten mit unerwünschtem Geruch durchgeführt wird.

Die zur Polymerisation eingesetzten Monomeren werden vorzugsweise zu wenigstens 95, besonders bevorzugt zu wenigstens 99 und insbesondere zu wenigstens 99,9% umgesetzt (Polymerisationsgrad).

Die für die erfindungsgemäße Verwendung geeigneten Polymere können aber auch durch andere übliche Polymerisationsverfahren wie beispielsweise Emulsionspolymerisation, Fällungspolymerisation, Wasser-in-Wasser-Emulsionspolymerisation, Polymerisation in Masse hergestellt werden. Diese Aufzählung ist allerdings weder abschließend noch vollständig und soll die Erfindung in keinster Weise einschränken.

Die nach der Polymerisation in Lösung oder dispergiert vorliegenden Polymere können durch übliche, dem Fachmann bekannte Trocknungsverfahren in Pulver überführt werden. Bevorzugte Verfahren sind die Sprühtrocknung, die Sprühwirbelschichttrocknung, die Walzentrocknung und die Bandtrocknung. Ebenfalls anwendbar sind die Gefriertrocknung und die Gefrierkonzentrierung.
Gewünschtenfalls können Lösungsmittel auch durch übliche Methoden, z. B. Destillation bei verringertem Druck, teilweise oder vollständig entfernt werden.

Erfindungsgemäß bevorzugt werden die vorstehend beschriebenen Polymere als Konditioniermittel, insbesondere in Haarpflegezubereitungen, oder als Haarfestigungsmittel, insbesondere in Haarpflegezubereitungen sowie als Verdickungsmittel verwendet.

Die erfindungsgemäß zu verwendenden Polymere können selbstverständlich auch in anderen technischen Gebieten eingesetzt werden. Als Beispiele für solche weiteren Anwendungen seien genannt Oil Recovery, Papierverarbeitung, Waschmittel, Anstrich, oder Lackierung.Ein weiterer Gegenstand der Erfindung sind kosmetische oder pharmazeutische Mittel, enthaltend
A) wenigstens ein erfindungsgemäß zu verwendendes Polymer und
B) wenigstens einen kosmetisch akzeptablen Träger.

Die erfindungsgemäßen Mittel weisen vorzugsweise einen kosmetisch oder pharmazeutisch akzeptablen Träger B) auf, der ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Die erfindungsgemäßen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.
Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.
Vorteilhafterweise werden diejenigen Öle, Fette und/oder Wachse gewählt, die auf Seite 28, Zeile 39 bis Seite 34, Zeile 22 der WO 2006/106140 beschrieben sind. Auf den Inhalt der genannten Textstelle wird hiermit in vollem Umfang Bezug genommen.
Der Gehalt an weiteren Ölen, Fetten und Wachsen beträgt höchstens 50, bevorzugt 30, weiter bevorzugt höchstens 20 Gewichts-%, bezogen auf das Gesamtgewicht der Zusammensetzung.

Geeignete hydrophile Träger B) sind ausgewählt unter Wasser, ein-, zwei- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden Eigenschaften eignen sich die zuvor beschriebenen Copolymere und Polyelektrolytkomplexe insbesondere als Zusatzstoffe für Haar- und Hautkosmetika.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes erfindungsgemäß zu verwendendes Polymer, wenigstens einen wie vorstehend definierten Träger B) und wenigstens einen davon verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Die erfindungsgemäßen kosmetischen Zusammensetzungen können als wässrige oder wässrig-alkoholische Lösungen, O/W- sowie W/O-Emulsionen, Hydrodispersionsformulierungen, feststoffstablilisierte Formulierungen, Stiftformulierungen, PIT-Formulierungen, in Form von Cremes, Schäumen, Sprays (Pumpspray oder Aerosol), Gelen, Gelsprays, Lotionen, Ölen, Ölgelen oder Mousse vorliegen und dementsprechend mit üblichen weiteren Hilfsstoffen formuliert werden.

Besonders bevorzugte kosmetische Zusammensetzungen im Sinne der vorliegenden Erfindung sind Shampoos und Haarpflegemittel. Die Erfindung betrifft demnach auch Zusammensetzungen zur Reinigung und/oder Pflege der Haare.

Insbesondere betrifft die Erfindung Haarpflegemittel ausgewählt aus der Gruppe bestehend aus Vorbehandlungsmitteln, Haarspülungen, Haarconditionern, Haarbalsamen, leave-on-Haarkuren, rinse-off-Haarkuren, Haarwässern, Pomaden, Frisiercremes, Frisierlotionen, Frisiergelen, Spitzenfluids, Hot-Oil-Treatments und Schaumkuren.

Weiterhin betrifft die Erfindung kosmetische Zusammensetzungen, die ausgewählt sind aus Gelcremes, Hydroformulierungen, Stiftformulierungen, kosmetischen Ölen und Ölgelen, Mascara, Selbstbräunern, Gesichtspflegemitteln, Körperpflegemitteln, After-Sun-Präparaten, Haarverformungsmitteln und Haarfestigern.

Weitere erfindungsgemäße kosmetische Zusammensetzungen sind hautkosmetische Zusammensetzungen, insbesondere solche zur Pflege der Haut. Diese liegen insbesondere als W/O- oder O/W-Hautcremes, Tag- und Nachtcremes, Augencremes, Gesichtscremes, Antifaltencremes, Mimikcremes, Feuchthaltecremes, Bleichcremes, Vitamincremes, Hautlotionen, Pflegelotionen und Feuchthaltelotionen vor.
Weiterhin eignen sich die erfindungsgemäßen Polymerkombinationen als Inhaltsstoffe für hautkosmetische Zubereitungen wie Gesichtswässer, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Makeup, Grundierungen, Rouges und Pudern und Augenbrauenstiften.

Außerdem können die erfindungsgemäßen Zusammensetzungen verwendet werden in Nose-Strips zur Porenreinigung, in Antiaknemitteln, Repellentien, Rasiermitteln, Haarentfernungsmitteln, Intimpflegemitteln, Fußpflegemitteln sowie in der Babypflege.

Weitere bevorzugte erfindungsgemäße Zusammensetzungen sind Wasch-, Dusch- und Badepräparate, die erfindungsgemäß zu verwendende Polymere enthalten.

Unter Wasch-, Dusch- und Badepräparaten werden im Rahmen dieser Erfindung Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes verstanden.
Geeignete weitere Inhaltsstoffe für diese erfindungsgemäßen Wasch-, Dusch- und Badepräparate sind im Folgenden beschrieben.

Die Zusammensetzungen enthalten neben den erfindungsgemäß zu verwendenden Polymeren weitere kosmetisch akzeptable Zusätze wie beispielsweise Emulgatoren und Co-Emulgatoren, Lösungsmittel, Tenside, Ölkörper, Konservierungsmittel, Parfümöle, kosmetische Pflege- und Wirkstoffe wie AHA-Säuren, Fruchtsäuren, Ceramide, Phytantriol, Collagen, Vitamine und Provitamine, beispielsweise Vitamin A, E und C, Retinol, Bisabolol, Panthenol, natürliche und synthetische Lichtschutzmittel, Naturstoffe, Trübungsmittel, Lösungsvermittler, Repellentien, Bleichmittel, Färbemittel, Tönungsmittel, Bräunungsmittel (z.B. Dihydroxyaceton), Mikropigmente wie Titanoxid oder Zinkoxid, Überfettungsmittel, Perlglanzwachse, Konsistenzgeber, Verdicker, Solubilisatoren, Komplexbildner, Fette, Wachse, Silikonverbindungen, Hydrotrope, Farbstoffe, Stabilisatoren, pH-Wert Regulatoren, Reflektoren, Proteine und Proteinhydrolysate (z.B. Weizen-, Mandel- oder Erbsenproteine), Ceramid, Eiweißhydrolysate, Salze, Gelbildner, Konsistenzgeber, Silikone, Feuchthaltemittel (z.B. 1,2-Pentandiol), Rückfetter, UV-Lichtschutzfilter und weitere übliche Additive. Des weiteren können zur Einstellung der jeweils gewünschten Eigenschaften insbesondere auch weitere Polymere enthalten sein.
Die erfindungsgemäßen kosmetischen Zusammensetzungen enthalten erfindungsgemäß zu verwendende Polymere in einer Menge von 0,01 bis 10 Gew.-%, bevorzugt 0,05 bis 5 Gew.-%, besonders bevorzugt 0,1 bis 1,5 Gew.-%, bezogen auf das Gewicht der Zusammensetzung.
In einer bevorzugten Ausführungsform der Erfindung enthalten die erfindungsgemäßen Wasch-, Dusch- und Badepräparate sowie Shampoos und Haarpflegemittel weiterhin wenigstens ein Tensid.
In einer weiteren bevorzugten Ausführungsform der Erfindung enthalten die erfindungsgemäßen Shampoos und Haarpflegemittel neben den erfindungsgemäß zu verwendenden Polymeren weiterhin wenigstens eine Öl- und/oder Fettphase und ein Tensid.

### Tenside

Als Tenside können anionische, kationische, nichtionische und/oder amphotere Tenside eingesetzt werden.

Vorteilhafte waschaktive anionische Tenside im Sinne der vorliegenden Erfindung sind
- Acylaminosäuren und deren Salze, wie Acylglutamate, insbesondere Natriumacylglutamat
- Sarcosinate, beispielsweise Myristoyl Sarcosin, TEA-Lauroyl Sarcosinat, Natriumlauroylsarcosinat und Natriumcocoylsarcosinat, Sulfonsäuren und deren Salze, wie
- Acylisethionate, beispielsweise Natrium- oder Ammoniumcocoylisethionat
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
- Alkylethersulfate, beispielsweise Natrium-, Ammonium-, Magnesium-, MIPA-, TIPA-Laurethsulfat, Natriummyrethsulfat und Natrium C₁₂₋₁₃ Parethsulfat,
- Alkyethersulfonate, beispielsweise Natrium C12-15 Pareth-15 Sulfonat
- Alkylsulfate, beispielsweise Natrium-, Ammonium- und TEA-Laurylsulfat. Weitere vorteilhafte anionische Tenside sind
- Taurate, beispielsweise Natriumlauroyltaurat und Natriummethylcocoyltaurat,
- Ether-Carbonsäuren, beispielsweise Natriumlaureth-13 Carboxylat und Natrium PEG-6 Cocamide Carboxylat, Natrium PEG-7-Olivenöl-Carboxylat
- Phosphorsäureester und Salze, wie beispielsweise DEA-Oleth-10 Phosphat und Dilaureth-4 Phosphat,
- Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
- Acylglutamate wie Di-TEA-palmitoylaspartat und Natrium Caprylic/Capric Glutamat,
- Acylpeptide, beispielsweise Palmitoyl hydrolysiertes Milchprotein, Natrium Cocoyl hydrolysiertes Sojaprotein und Natrium-/Kalium Cocoyl hydrolysiertes Kollagen sowie Carbonsäuren und Derivate, wie beispielsweise Laurinsäure, Aluminiumstearat, Magnesiumalkanolat und Zinkundecylenat, Ester-Carbonsäuren, beispielsweise Calciumstearoyllactylat, Laureth-6 Citrat und Natrium PEG-4 Lauramidcarboxylat
- Alkylarylsulfonate.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.
Weitere vorteilhafte kationische Tenside im Sinne der vorliegenden Erfindung sind ferner
- Alkylamine,
- Alkylimidazole und
- ethoxylierte Amine
und insbesondere deren Salze.

Vorteilhafte waschaktive amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.
Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.
Vorteilhafte waschaktive nicht-ionische Tenside im Sinne der vorliegenden Erfindung sind
- Alkanolamide, wie Cocamide MEA/DEA/MIPA,
- Ester, die durch Veresterung von Carbonsäuren mit Ethylenoxid, Glycerin, Sorbitan oder anderen Alkoholen entstehen,
- Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil^{®}SPG 128V (Wacker)).
Weitere vorteilhafte nicht-ionische Tenside sind Alkohole und Aminoxide, wie Cocoamidopropylamin-Oxid.

Bevorzugte anionische, amphotere und nichtionische Shampoo-Tenside sind beispielsweise in "Kosmetik und Hygiene von Kopf bis Fuß", Hrsg. W. Umbach, 3. Auflage, Wiley-VCH, 2004, S.131-134 genannt, worauf an dieser Stelle in vollem Umfang Bezug genommen wird.
Unter den Alkylethersulfaten sind insbesondere Natriumalkylethersulfate auf Basis von zwei- oder dreifach ethoxyliertem Lauryl- und Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Haut- und Schleimhautverträglichkeit. Sie können auch als alleinige Waschrohstoffe für Shampoos eingesetzt werden können. Laurylethersulfat weist bessere Schaumeigenschaften als Myristylethersulfat auf, ist diesem aber in der Milde unterlegen.
Alkylethercarboxylate werden oft in Kombination mit Alkylethersulfaten und amphoteren Tensiden in Haarwaschmitteln eingesetzt.
Sulfobernsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt. Amidopropylbetaine sind als alleinige Waschrohstoffe praktisch unbedeutend, da ihr Schaumverhalten sowie ihre Verdickbarkeit nur mäßig ausgeprägt sind. Demgegenüber weisen diese Tenside eine ausgezeichnete Haut- und Augenschleimhautverträglichkeit auf. In Kombination mit anionischen Tensiden lässt sich deren Milde synergistisch verbessern. Bevorzugt ist die Verwendung von Cocamidopropylbetain.

Amphoacetate /Amphodiacetate besitzen als amphotere Tenside eine sehr gute Haut-und Schleimhautverträglichkeit und können haarkonditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.
Alkylpolyglykoside sind nichtionische Waschrohstoffe. Sie sind mild, haben gute Universaleigenschaften, schäumen jedoch schwach. Aus diesem Grund werden sie bevorzugt in Kombinationen mit anionischen Tensiden verwendet.
Sorbitanester gehören ebenfalls zu den nichtionischen Waschrohstoffen. Wegen ihrer ausgezeichneten Milde werden sie bevorzugt zum Einsatz bei Baby-Shampoos verwendet. Als Schwachschäumer werden sie bevorzugt in Kombination mit anionischen Tensiden eingesetzt.
Es ist vorteilhaft, das oder die waschaktiven Tenside aus der Gruppe der Tenside zu wählen, welche einen HLB-Wert von mehr als 25 haben, besonders vorteilhaft sind solche, welchen einen HLB-Wert von mehr als 35 haben.
Es ist erfindungsgemäß von Vorteil, wenn ein oder mehrere dieser Tenside in einer Konzentration von 1 bis 30 Gewichts-%, bevorzugt in einer Konzentration von 5 bis 25 Gewichts-% und ganz besonders bevorzugt in einer Konzentration von 10 bis 20 Gewichts-%, jeweils bezogen auf das Gesamtgewicht der Zusammensetzung eingesetzt werden.

### Polysorbate

Als waschaktive Agentien können auch Polysorbate vorteilhaft in die erfindungsgemäßen Zusammensetzungen eingearbeitet werden. Im Sinne der Erfindung vorteilhafte Polysorbate sind beispielsweise
- Polyoxyethylen(20)sorbitanmonolaurat (Tween^{®}20, CAS-Nr. 9005-64-5)
- Polyoxyethylen(4)sorbitanmonolaurat (Tween^{®}21, CAS-Nr. 9005-64-5)
- Polyoxyethylen(4)sorbitanmonostearat (Tween^{®}61, CAS-Nr. 9005-67-8)
- Polyoxyethylen(20)sorbitantristearat (Tween^{®}65, CAS-Nr. 9005-71 -4)
- Polyoxyethylen(20)sorbitanmonooleat (Tween^{®}80, CAS-Nr. 9005-65-6)
- Polyoxyethylen(5)sorbitanmonooleat (Tween^{®}81, CAS-Nr. 9005-65-5)
- Polyoxyethylen(20)sorbitantrioleat (Tween^{®}85, CAS-Nr. 9005-70-3). Besonders vorteilhaft sind
- Polyoxyethylen(20)sorbitanmonopalmitat (Tween^{®}40, CAS-Nr. 9005-66-7) und
- Polyoxyethylen(20)sorbitanmonostearat (Tween^{®}60, CAS-Nr. 9005-67-8).
   Die Polysorbate werden vorteilhaft in einer Konzentration von 0,1 bis 5 und insbesondere in einer Konzentration von 1,5 bis 2,5 Gew.-%, bezogen auf das Gesamtgewicht der Zusammensetzung einzeln oder als Mischung mehrer Polysorbate, eingesetzt.

### Weitere Konditionierungsmittel

Sofern gewünscht, enthalten die erfindungsgemäßen Zusammensetzungen zusätzlich zu den erfindungsgemäß zu verwendenden Polymeren weitere Konditionierungsmittel. Als weitere Konditionierungsmittel für die erfindungsgemäßen kosmetischen Zusammensetzungen werden bevorzugt diejenigen Konditionierungsmittel gewählt, die auf Seite 34, Zeile 24 bis Seite 37, Zeile 10 der WO 2006/106140 beschrieben sind. Auf den Inhalt der genannten Textstelle wird hiermit in vollem Umfang Bezug genommen.

### Rheologiemodifizierungsmittel

Bei den geeigneten Rheologiemodifizierungsmitteln handelt es sich vor allem um Verdicker. Für Shampoos und Haarpflegemittel geeignete Verdicker sind in "Kosmetik und Hygiene von Kopf bis Fuß", Hrsg. W. Umbach, 3. Auflage, Wiley-VCH, 2004, S.235-236 genannt, worauf an dieser Stelle vollumfänglich Bezug genommen wird. Geeignete Verdickungsmittel für die erfindungsgemäßen kosmetischen Zusammensetzungen sind beispielsweise auch auf Seite 37, Zeile 12 bis Seite 38, Zeile 8 der WO 2006/106140 beschrieben. Auf den Inhalt der genannten Textstelle wird hiermit in vollem Umfang Bezug genommen.

### Konservierungsmittel

Die erfindungsgemäßen kosmetischen Zusammensetzungen können auch Konservierungsmittel enthalten. Zusammensetzungen mit hohen Wassergehalten müssen zuverlässig vor Verkeimung geschützt sein. Geeignete Konservierungsmittel für die erfindungsgemäßen kosmetischen Zusammensetzungen sind beispielsweise auf Seite 38, Zeile 10 bis Seite 39, Zeile 18 der WO 2006/106140 beschrieben. Auf den Inhalt der genannten Textstelle wird hiermit in vollem Umfang Bezug genommen.

Komplexbildner: Da die Rohstoffe und auch die kosmetischen Mittel selbst häufig überwiegend in Stahlapparaturen hergestellt werden, können die Endprodukte Eisen(-Ionen) in Spurenmengen enthalten. Um zu verhindern, dass diese Verunreinigungen über Reaktionen mit Farbstoffen und Parfümölbestandteilen die Produktqualität nachteilig beeinflussen, werden Komplexbildner wie Salze der Ethylendiamintetraessigsäure, der Nitrilotriessigsäure, der Iminodibernsteinsäure oder Phosphate zugesetzt.

UV- Lichtschutzfilter: Um die in den erfindungsgemäßen kosmetischen Zusammensetzungen enthaltenen Inhaltsstoffe wie beispielsweise Farbstoffe und Parfümöle gegen Veränderungen durch UV-Licht zu stabilisieren, können UV- Lichtschutzfilter, wie z. B. Benzophenon-Derivate, eingearbeitet werden. Geeignete UV- Lichtschutzfilter für die erfindungsgemäßen kosmetischen Zusammensetzungen sind beispielsweise auf Seite 39, Zeile 20 bis Seite 41 Zeile 10 der WO 2006/106140 beschrieben. Auf den Inhalt der genannten Textstelle wird hiermit in vollem Umfang Bezug genommen.

Antioxidantien: Ein Gehalt der erfindungsgemäßen kosmetischen Zusammensetzungen an Antioxidantien ist im allgemeinen bevorzugt. Erfindungsgemäß können als Antioxidantien alle für kosmetische Anwendungen geeigneten oder gebräuchlichen Antioxidantien verwendet werden. Geeignete Antioxidantien für die erfindungsgemäßen kosmetischen Zusammensetzungen sind beispielsweise auf Seite 41, Zeile 12 bis Seite 42 Zeile 33 der WO 2006/106140 beschrieben. Auf den Inhalt der genannten Textstelle wird hiermit in vollem Umfang Bezug genommen.

Puffer: Puffer gewährleisten die pH-Wert-Stabilität der kosmetischen Zusammensetzungen. Überwiegend verwendet werden Citrat-, Lactat- und Phosphat-Puffer.

Lösungsvermittler: Sie werden eingesetzt, um pflegende Öle oder Parfümöle klar in Lösung zu bringen und auch in der Kälte klar in Lösung zu halten. Die gängigsten Lösungsvermittler sind ethoxylierte nichtionische Tenside, z. B. hydrierte und ethoxylierte Ricinusöle.

Keimhemmende Mittel: Weiterhin können auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid). Quartäre Ammonium-Verbindungen sind prinzipiell ebenfalls geeignet und werden bevorzugt für desinfizierende Seifen und Waschlotionen verwendet. Auch zahlreiche Riechstoffe haben antimikrobielle Eigenschaften. Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit.
Die antibakteriell wirksamen Stoffe werden in der Regel in Konzentrationen von ca. 0,1 bis 0,3 Gew.-% eingesetzt.

Dispergiermittel: Wenn in den erfindungsgemäßen kosmetischen Zusammensetzungen unlösliche Wirkstoffe, z.B. Antischuppenwirkstoffe oder Siliconöle, dispergiert und auf Dauer in Schwebe gehalten werden sollen, müssen Dispergiermittel und Verdicker wie z. B. Magnesium-Aluminium-Silicate, Bentonite, Fettacyl-Derivate, Polyvinylpyrrolidon oder Hydrokolloide, z. B. Xanthan Gum oder Carbomere, eingesetzt werden. Erfindungsgemäß sind Konservierungsmittel in einer Gesamtkonzentration von höchstens 2, bevorzugt höchstens 1,5 und besonders bevorzugt höchstens 1 Gewichts-%, bezogen auf das Gesamtgewicht der Zusammensetzung, enthalten.

Die kosmetischen Zusammensetzungen können außer den vorgenannten Substanzen gegebenenfalls die in der Kosmetik üblichen Zusatzstoffe, beispielsweise Parfüm, Farbstoffe, rückfettende Agentien, Komplexierungs- und Sequestrierungsagentien, Perlglanzagentien, Pflanzenextrakte, Vitamine, Wirkstoffe, Pigmente, die eine färbende Wirkung haben, weichmachende, anfeuchtende und/oder feuchthaltende Substanzen, oder andere übliche Bestandteile einer kosmetischen oder dermatologischen Formulierung wie Alkohole, Polyole, Polymere, organische Säuren zur pH-Wert-Einstellung, Schaumstabilisatoren, Elektrolyte, organische Lösemittel oder Silikonderivate enthalten.
Hinsichtlich der genannten, dem Fachmann bekannten weiteren Inhaltsstoffe für die Zusammensetzungen sei auf "Kosmetik und Hygiene von Kopf bis Fuß", Hrsg. W. Umbach, 3. Auflage, Wiley-VCH, 2004, S.123-128 verwiesen, worauf an dieser Stelle vollumfänglich Bezug genommen wird.

Die erfindungsgemäß zu verwendenden Polymere sind, insbesondere in kationischer Form, geeignet, die Abscheidungsmenge und -geschwindigkeit sowie die Verweilzeit von weiteren Wirkstoffen, wie beispielsweise Silikonen oder UV-Lichtschutzfiltern, auf der Haut und/oder dem Haar zu verstärken bzw. zu erhöhen. Substanzen oder Mittel, die solche Effekte besitzen, werden auch als Depositioning Aids bezeichnet.

US 6,998,113 beschreibt rinse-off-Zubereitungen, die dazu führen, dass die damit behandelte Haut effektiv vor UV-Strahlung geschützt wird. Einige der dort beschriebenen Zubereitungen enthalten kationische Polymere. Im Sinne der vorliegenden Erfindung können auch die erfindungsgemäßen Copolymere in den Zubereitungen der US 6,998,113 verwendet werden. Insbesondere können die erfindungsgemäß zu verwendenden Polymere für den von der US 6,998,113 genannten Zweck in Sonnenschutz-Wasch- und Badepräparaten eingesetzt werden. Auf die Offenbarung der US 6,998,113 wird hiermit in vollem Umfang Bezug genommen.

Geeignete Silikone sind beispielsweise in der US 5,935,561, Spalte 13, Z.64 bis Spalte 18, Z.61 dargestellt, worauf hiermit in vollem Umfang Bezug genommen wird.

Stellvertretend seien genannt:
- Dimethicone
- Polyalkyl -oder Polyarylsiloxane (US 5,935,561, Spalte 13, Formel I)
- Alkylamino substituierte Silikone (US 5,935,561, Spalte 14, Formel II (Amodimethicone))
- Kationische Silikone (US 5,935,561, Spalten 14 und 15, Formel III)
- Trimethylsilylamodimethicone (US 5,935,561, Spalte 15, Formel IV)
- Silikone gemäß US 5,935,561, Spalte 15, Formel V
- cyclische Polysiloxane gemäß US 5,935,561, Spalte 16, Formel VI
- Ethoxylierte Glycerin-Fettsäureester

Die erfindungsgemäßen kosmetischen Zusammensetzungen wie Shampoos und Haarpflegemittel enthalten gegebenenfalls ethoxylierte Öle ausgewählt aus der Gruppe der ethoxylierten Glycerin-Fettsäureester, insbesondere bevorzugt PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fett-Säuren, PEG-26 Jojobaalkohol), Glycereth-5 Cocoat, PEG-9 Kokosfettsäureglyceride, PEG-7 Glycerylcocoat, PEG-45 Palmkernölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG- 18 Glyceryloleat-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat.
Bevorzugte ethoxylierte Öle sind PEG-7 Glycerylcocoat, PEG-9 Kokosglyceride, PEG-40 Hydriertes Rizinusöl, PEG-200 hydriertes Glycerylpalmat.
Ethoxylierte Glycerin-Fettsäureester werden in wässrigen Reinigungsrezepturen zu verschiedenen Zwecken eingesetzt. Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt.

### Wi rkstoffe

In die erfindungsgemäßen kosmetischen Zusammensetzungen lassen sich verschiedenste Wirkstoffe mit unterschiedlicher Löslichkeit homogen einarbeiten. Vorteilhafte Wirkstoffe in den erfindungsgemäßen kosmetischen Zusammensetzungen sind beispielsweise auf Seite 44, Zeile 24 bis Seite 49 Zeile 39 der WO 2006/106140 beschrieben. Auf den Inhalt der genannten Textstelle wird hiermit in vollem Umfang Bezug genommen.

### UV-Lichtschutzmittel

Die erfindungsgemäßen kosmetischen Zusammensetzungen enthalten in einer bevorzugten Ausführungsform UV-Lichtschutzmittel zum Schutz der Haut und/oder der Haare. Geeignete UV-Lichtschutzmittel sind in der WO 2006/106114, S.24, Z.4 bis S.27, Z.27 ausführlich beschrieben, worauf hiermit in vollem Umfang Bezug genommen wird. Vorteilhaft enthalten die kosmetischen Zusammensetzungen Substanzen, die UV-Strahlung im UVB-Bereich und Substanzen, die UV-Strahlung im UVA-Bereich absorbieren, wobei die Gesamtmenge der Filtersubstanzen z.B. 0,1 bis 30 Gew.-%, vorzugsweise 0,5 bis 20 Gew.-%, insbesondere 1 bis 15 Gew.-% beträgt, bezogen auf das Gesamtgewicht der Zusammensetzungen, um kosmetische Zusammensetzungen zur Verfügung zu stellen, welche die Haut vor dem gesamten Bereich der ultravioletten Strahlung schützen.
Der größte Teil der Lichtschutzmittel in den zum Schutz der menschlichen Epidermis dienenden kosmetischen oder dermatologischen Zusammensetzungen besteht aus Verbindungen, die UV-Licht im UV-B-Bereich absorbieren. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

### Perlglanzwachse

Geeignete Perlglanzwachse für die erfindungsgemäßen kosmetischen Zusammensetzungen sind beispielsweise auf Seite 50, Zeile 1 bis Zeile 16 der WO 2006/106140 beschrieben. Auf den Inhalt der genannten Textstelle wird hiermit in vollem Umfang Bezug genommen.
Die erfindungsgemäßen kosmetischen Zusammensetzungen können weiterhin Glitterstoffe und/oder andere Effektstoffe (z.B. Farbschlieren) enthalten.

### Emulgatoren

Die erfindungsgemäßen kosmetischen Zusammensetzungen liegen in einer bevorzugten Ausführungsform der Erfindung in Form von Emulsionen vor. Die Herstellung solcher Emulsionen erfolgt nach bekannten Methoden. Geeignete Emulgatoren für die erfindungsgemäßen Emulsionen sind beispielsweise auf Seite 50, Zeile 18 bis Seite 53, Zeile 4 der WO 2006/106140 beschrieben. Auf den Inhalt der genannten Textstelle wird hiermit in vollem Umfang Bezug genommen.

### Parfümöle

Sollen den erfindungsgemäßen kosmetischen Zusammensetzungen Parfumöle zugesetzt werden, so sind geeignete Parfümöle beispielsweise auf Seite 53, Zeile 10 bis Seite 54, Zeile 3 der WO 2006/106140 beschrieben. Auf den Inhalt der genannten Textstelle wird hiermit in vollem Umfang Bezug genommen.

### Pigmente

Gegebenenfalls enthalten die erfindungsgemäßen kosmetischen Zusammensetzungen weiterhin Pigmente. Die Pigmente liegen im Produkt meist in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 µm, insbesondere 3 bis 150 µm, besonders bevorzugt 10 bis 100 µm.
Geeignete Pigmente für die erfindungsgemäßen Zusammensetzungen sind beispielsweise auf Seite 54, Zeile 5 bis Seite 55, Zeile 19 der WO 2006/106140 beschrieben. Auf den Inhalt der genannten Textstelle wird hiermit in vollem Umfang Bezug genommen.

### Polymere

Die erfindungsgemäßen kosmetischen Zusammensetzungen enthalten in einer bevorzugten Ausführungsform außer den erfindungsgemäß zu verwendenden Polymeren weitere Polymere.
Geeignete zusätzliche Polymere für die erfindungsgemäßen Zusammensetzungen sind beispielsweise auf Seite 55, Zeile 21 bis Seite 63, Zeile 2 der WO 2006/106140 beschrieben. Auf den Inhalt der genannten Textstelle wird hiermit in vollem Umfang Bezug genommen.

### Shampoo-Typen

Eine bevorzugte Ausführungsform der Erfindung sind Haar-Shampoos enthaltend die erfindungsgemäßen Copolymere. An Shampoos werden je nach Haarqualität oder Kopfhautproblem gegebenenfalls zusätzliche Anforderungen gestellt. Die Wirkungsweise der bevorzugten Shampoo-Typen mit den wichtigsten zusätzlichen Wirkungen bzw. wichtigsten speziellen Zielsetzungen wird nachfolgend beschrieben. Erfindungsgemäß bevorzugt sind beispielsweise Shampoos für normales bzw. schnell fettendes bzw. geschädigtes Haar, Antischuppenshampoos, Babyshampoos und 2-in-1-Shampoos (also Shampoo und Spülung in einem).
Erfindungsgemäße Shampoos für normales Haar: die Haarwäsche soll Haar und Kopfhaut von dem in Talgdrüsen gebildeten Hautfett, den aus Schweissdrüsen mit Wasser austretenden anorganischen Salzen, Aminosäuren, Harnstoff und Milchsäure, abgeschilferten Hautpartikeln, Umweltschmutz, Gerüchen und gegebenenfalls Rückständen haarkosmetischer Behandlungen befreien. Normales Haar bedeutet kurzes bis schulterlanges Haar, welches nur schwach geschädigt ist. Dementsprechend soll der Anteil an Konditionierhilfsstoffen auf diesen Haartyp optimiert sein.
Erfindungsgemäße Shampoos für schnell fettendes Haar: eine erhöhte Fettproduktion der Talgdrüsen der Kopfhaut führt bereits 1-2 Tage nach der Haarwäsche zu einer strähnigen, unansehnlichen Frisur. Öl- und wachsartige Hautfettbestandteile beschweren das Haar und mindern die Reibung von Haar zu Haar und verringern damit den Frisurenhalt. Das eigentliche haarkosmetische Problem bei schnell fettenden Haaren ist also das vorzeitige Zusammenfallen voluminöser Frisuren. Um dies zu vermeiden, muss man verhindern, dass die Haaroberfläche beschwert und zu glatt und geschmeidig wird. Dies wird bevorzugt durch die Tensidgrundlage aus gut reinigenden und besonders wenig substantiv ausgeprägten Waschrohstoffen erreicht. Zusätzliche Pflegestoffe, die sich zum Hautfett addieren würden, wie rückfettende Substanzen werden in Shampoos für schnell fettendes Haar nicht oder nur mit größter Vorsicht eingesetzt. Erfindungsgemäße volumengebende Shampoos für feines Haar können vergleichbar formuliert werden.
Erfindungsgemäße Shampoos für trockenes, strapaziertes (geschädigtes) Haar: Die Struktur des Haares wird im Laufe des Haarwachstums durch mechanische Einflüsse wie Kämmen, Bürsten und vor allen Dingen Toupieren (Kämmen gegen die Wuchsrichtung), durch die Einwirkung von UV-Strahlung bzw. sichtbarem Licht und durch kosmetische Behandlungen, wie Dauerwellen, Blondierung oder Färbung verändert. Die Schuppenschicht der Haare weist von der Wurzel bis zur Spitze ein zunehmend strapazierteres Aussehen auf; in Extremfällen ist sie an der Spitze völlig abgetragen, und die Haarspitzen sind gespalten (Haarspliss). Geschädigtes Haar kann grundsätzlich nicht mehr in den Zustand des gesunden Haarnachwuchses zurückgeführt werden. Es gelingt aber, diesem Idealzustand hinsichtlich Griff, Glanz und Kämmbarkeit durch Verwendung von erfindungsgemäßen Shampoos mit gegebenenfalls hohen Anteilen an pflegenden Stoffen (Konditioniermitteln) sehr nahe zu kommen.
Eine noch bessere haarkonditionierende Wirkung als mit einem Shampoo wird mit einem erfindungsgemäßen Haarpflegemittel beispielsweise in Form einer Spülungs- oder Kurmittelbehandlung nach der Haarwäsche erreicht. Spülungs- oder Kurmittel für Haare, die erfindungsgemäß zu verwendende Polymere enthalten, sind ebenfalls erfindungsgemäß.
Erfindungsgemäße 2-in-1- Shampoos sind besonders stark pflegende Shampoos, bei denen durch die Konzipierung als "Shampoo und Spülung in einem" der Zusatznutzen Pflege gleichwertig neben den Grundnutzen Reinigung gestellt wird. Erfindungsgemäße 2-in-1-Zusammensetzungen enthalten erhöhte Mengen an Konditioniermitteln. Antischuppenshampoos: Erfindungsgemäße Antischuppenshampoos haben im Vergleich zu Antischuppenhaarwässern den Vorteil, dass sie nicht nur durch entsprechende Wirkstoffe gegen Schuppenbefall die Bildung neuer sichtbarer Schuppen verringern und bei langfristiger Anwendung verhindern, sondern mit der Haarwäsche auch bereits abgeschilferte Schuppen entfernen. Nach dem Ausspülen der Waschflotte bleibt allerdings nur ein geringer, jedoch ausreichender Anteil der Wirkstoffe auf Kopfhaut und Haar zurück. Es gibt verschiedene Antischuppen-Wirkstoffe, die in die erfindungsgemäßen Shampoo-Zusammensetzungen eingearbeitet werden können, wie z. B. Zinkpyrithion, Ketokonazol, Elubiol, Clotrimazol, Climbazol oder Pirocton Olamin. Zusätzlich weisen diese Substanzen eine die Abschilferung normalisierende Wirkung auf.
Die Grundlage von Antischuppenshampoos entspricht überwiegend der Formulierung von Shampoos für normales Haar mit gutem Reinigungseffekt.
Babyshampoos: bei einer bevorzugten Ausführungsform der Erfindung handelt es sich bei den erfindungsgemäßen Shampoo-Zubereitungen um Babyshampoos. Diese sind optimal haut- und schleimhautverträglich. Kombinationen von Waschrohstoffen mit sehr guter Hautverträglichkeit bilden die Basis dieser Shampoos. Zusätzliche Stoffe zur weiteren Verbesserung der Haut- und Schleimhautverträglichkeit und der Pflegeeigenschaften werden vorteilhaft zugefügt, wie z. B. nichtionische Tenside, Proteinhydrolysate und Panthenol oder Bisabolol. Alle notwendigen Rohstoffe und Hilfsstoffe, wie Konservierungsmittel, Parfümöle, Farbstoffe usw., werden unter dem Aspekt einer hohen Verträglichkeit und Milde ausgewählt.

Shampoos für trockene Kopfhaut: bei einer weiteren bevorzugten Ausführungsform der Erfindung handelt es sich bei den erfindungsgemäßen Shampoo-Zubereitungen um Shampoos für trockene Kopfhaut. Das primäre Ziel dieser Shampoos ist die Verhinderung der Austrocknung der Kopfhaut, da trockene Kopfhaut zu Juckreiz, Rötung und Entzündung führen kann. Wie auch bei den Babyshampoos bilden Kombinationen von Waschrohstoffen mit sehr guter Hautverträglichkeit die Basis dieser Shampoos. Zusätzlich können gegebenenfalls Rückfetter und Feuchthaltemittel, wie z. B. Glycerin oder Harnstoff, eingesetzt werden.
Die erfindungsgemäßen Shampoo-Zusammensetzungen können auch als Shampookonzentrate mit erhöhten Tensidgehalten von 20-30 Gew.-% vorliegen. Sie basieren auf speziellen Waschrohstoffkombinationen und Konsistenzregulatoren, die die gute Verteilbarkeit und das spontane Anschäumvermögen auch einer kleinen Anwendungsmenge gewährleisten. Ein besonderer Vorteil ist beispielsweise die Möglichkeit, die Ergiebigkeit von 200 ml Shampoo mit einer 100-ml-Flasche zu erreichen.

### Darreichung

Es ist vorteilhaft, wenn die erfindungsgemäßen Zusammensetzungen in einer Flasche oder Quetschflasche aufbewahrt und aus dieser heraus angewendet werden. Demzufolge sind auch Flaschen oder Quetschflaschen, welche eine erfindungsgemäße Zusammensetzung enthalten, erfindungsgemäß.

Die erfindungsgemäßen Copolymere, wie zuvor definiert, können bevorzugt in Shampooformulierungen insbesondere als Konditioniermittel eingesetzt werden. Bevorzugte Shampooformulierungen enthalten
a) 0,05 bis 10 Gew.-% wenigstens eines erfindungsgemäßen Copolymers,
b) 25 bis 94,95 Gew.-% Wasser,
c) 5 bis 50 Gew.-% Tenside,
d) 0 bis 5 Gew.-% eines weiteren Konditioniermittels,
e) 0 bis 10 Gew.-% weitere kosmetische Bestandteile.

In den Shampooformulierungen können alle in Shampoos üblicherweise eingesetzten anionischen, neutralen, amphoteren oder kationischen Tenside verwendet werden. Geeignete Tenside wurden vorstehend genannt.

### Seifen und Syndets

Weitere erfindungsgemäße Zusammensetzungen, die die erfindungsgemäßen Copolymere enthalten, sind beispielsweise Seifen und Syndets.

Seife entsteht bei der Reaktion eines (Neutral-)Fettes oder daraus gewonnener Fettsäuren bzw. Fettsäuremethylester mit Natron- oder Kalilauge (Verseifung"). Seife ist in der Zusammensetzung chemisch das Alkalisalz von Fettsäuren. Als Neutralfette werden üblicherweise Rindertalg oder Palmöl im Gemisch mit Kokosöl oder Palmkernöl und - seltener - andere natürliche Öle oder Fette bei der Seifenherstellung eingesetzt, wobei die Qualität der Ausgangsfette stark mitbestimmend für die Güte der daraus gewonnenen Seife ist.
Wichtig für die Auswahl der Fettkomponenten ist die Verteilung der Kettenlängen der entsprechenden Fettsäuren. Gefragt sind normalerweise vor allem C12-C18-Fettsäuren. Da Lauratseife besonders gut schäumt, werden üblicherweise das laurinreiche Kokosöl oder das ähnlich aufgebaute Palmkernöl in höheren Anteilen (bis zu 50% des Neutralfettgemisches) für Seifen verwendet, bei denen viel Schaum bei der Benutzung erwünscht ist.
Die Natriumsalze der genannten Fettsäuregemische sind fest, die Kaliumsalze dagegen weich und pastös. Aus diesem Grunde wird als Laugenkomponente zur Herstellung fester Seifen vorzugsweise Natronlauge, für flüssig-pastöse Seifen vorzugsweise Kalilauge verwendet. Bei der Verseifung wird das Verhältnis von Lauge zu Fettsäure so gewählt, dass allenfalls ein minimaler Überschuss an Lauge (max.0,05 %) im fertigen Seifenstück vorhanden ist.

Zu den Seifen werden üblicherweise Toilette-, Kern-, Transparent-, Luxus-, Creme-, Frische-/Deo-, Baby-, Hautschutz-, Abrasiv-, Schwimm- und Flüssigseifen sowie Waschpasten und Seifenblättchen gezählt.

Erfindungsgemäße Seifen enthalten neben den erfindungsgemäß zu verwendenden Polymeren vorteilhafterweise weiterhin Antioxidantien, Komplexierungs- und Feuchthaltemittel sowie gegebenenfalls Duftstoffe, Farbstoffe und weitere, kosmetisch akzeptable Inhaltsstoffe. Solche weiteren geeigneten Inhaltsstoffe sind vorstehend genannt.

Syndets (von engl. synthetic detergent) sind Alternativen zu den herkömmlichen Seifen, die durch die gegenüber der Seife unterschiedliche Zusammensetzung gewisse Vorteile aufweist, wo Seife eher Nachteile besitzt.

Syndets enthalten als Schaum- und Reinigungskomponenten waschaktive Substanzen (Tenside), die durch chemische Synthese gewonnen werden. Seifen dagegen sind - wie beschrieben - Salze natürlich vorkommender Fettsäuren. Für Syndets werden hautmilde, biologisch gut abbaubare Tenside verwendet, vorzugsweise Fettsäure-Isethionate (Sodium Cocoyl Isethionate), Sulfobernsteinsäure-Halbester (Disodium Lauryl Sulfosuccinate), Alkylpolyglucoside (Decyl Glucoside), Amphotertenside (z. B. Sodium Cocoamphoacetate). Daneben spielen Monoglycerid-Sulfat und Ethercarboxylate vereinzelt eine Rolle. Fettalkoholsulfat (z. B. Sodium Lauryl Sulfate) hat seine einstige Bedeutung als Basistensid für Syndets weitestgehend verloren. Die Basistenside werden mit Gerüstsubstanzen, Rückfettungsmitteln und weiteren Zusätzen zu Formulierungen kombiniert, die sich nach üblicher Seifentechnologie verarbeiten lassen und Stücke ergeben, die sich möglichst "seifenähnlich", aber ohne die erwähnten Nachteile der Seife verhalten. Sie schäumen bei jeder Wasserhärte und besitzen eine sehr gute Reinigungskraft. Ihr pH-Wert ist in einem breiten Bereich (meist zwischen 4 und 8) einstellbar.
Aufgrund der intensiveren Reinigungs-/Entfettungskraft der Basistenside ist der Tensidanteil im Syndet gewöhnlich deutlich geringer, der Anteil an Überfettungsmitteln deutlich höher als in Seifen, ohne dass das Schaumvermögen herabgesetzt wird. Syndets werden speziell zur Reinigung der sensiblen Haut, der jugendlich-unreinen Haut und zur Gesichtswäsche empfohlen.

Neben den (seifenfreien) Syndets findet man auch das Marktsegment der Halb- oder Combars (abgeleitet von Combination bar). Dabei handelt es sich um Stücke, die sowohl Seife als auch Syndettenside enthalten. Combars enthalten 10 bis 80 Gew.-% Seife. Sie stellen für die Kriterien Kosten, Schaumvermögen, Hautgefühl und Verträglichkeit einen Kompromiss zwischen Seifen und Syndets dar. Beim Waschen mit einem Combar stellt sich, abhängig von seinem Seifenanteil, ein pH-Wert von ca. 7 bis 9 ein.

Hinsichtlich dem Fachmann bekannter, möglicher Rezepturen für Seifen und Syndets sei auf "Kosmetik und Hygiene von Kopf bis Fuß", Hrsg. W. Umbach, 3. Auflage, Wiley-VCH, 2004, S.112-122 verwiesen, worauf an dieser Stelle vollumfänglich Bezug genommen wird.

### Duschbad- und Badeprodukte

Hinsichtlich spezieller Zusammensetzungen für Duschbad- und Badeprodukte oder Waschlotionen sei auf "Kosmetik und Hygiene von Kopf bis Fuß", Hrsg. W. Umbach, 3. Auflage, Wiley-VCH, 2004, S.128-134 verwiesen, worauf an dieser Stelle vollumfänglich Bezug genommen wird.

Ein weiterer Gegenstand der Erfindung ist die Verwendung der zuvor beschriebenen Polymere als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

### Beispiele

Die folgenden Beispiele sollen die Erfindung näher erläutern ohne sie darauf zu beschränken

### Abkürzungen

- AS: Acrylsäure
- DADMAC: Diallyldimethylammoniumchlorid
- EA: Ethylacetat
- ES: Essigsäure
- EtOH: Ethanol
- ImEMA: 2-(1-Imidazolyl)ethylmethacrylat
- MAM: Methylmethacrylamid
- tBPPiv: tert.-butyl Perpivalat
- Wako®V50: 2,2'-azobis(2-amidinopropan)dihydrochlorid
- VP: N-Vinylpyrrolidon
- VI: N- Vinylimidazol

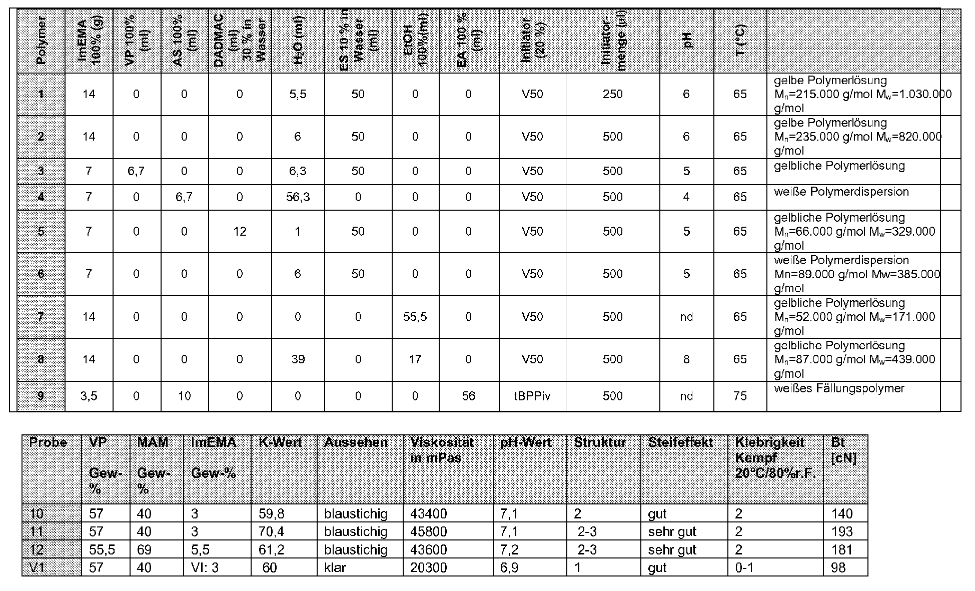

Die Polymere 1 bis 9 der obenstehenden Tabelle 2 wurden in Batch-Fahrweise in einer Workstation Modell A100 der Fa. Chemspeed®Technologies hergestellt. Der Reaktionsansatz betrug jeweils ca. 70 ml.

### ImEMA/DADMAC-Copolymer (Polymer 5)

Zur Herstellung eines P(ImEMA-co-DADMAC) Copolymers wurden 7 g ImEMA und 12 ml einer 30 Gew.-%igen wässrigen DADMAC-Lösung gemischt. Zusätzlich wurden 50 ml 10 Gew.-%ige Essigsäure und 1 ml Wasser zugefügt. Danach wurden 500 ml einer 20 Gew.-%igen wässrigen Wako®V50-Initiator-Lösung zugegeben und die Reaktionsmischung auf 65°C erwärmt und während 3 Stunden polymerisiert. Das resultierende Copolymer wies ein durch Size Exclusion Chromatography (Kalibrierung mit eng verteilten Pullulan ( = lineare Polymaltotriose ) -Standards (Fa. PSS, Deutschland) mit Molekulargewichten von Mₙ= 5 600 bis Mₙ= 710 000 g/mol, sowie Maltohexaose (Mₙ=992 g/mol);ausserhalb dieses Intervalls liegende Elutionsbereiche wurden durch Extrapolation geschätzt) bestimmtes zahlenmittleres Molekulargewicht von Mₙ = 66.000 g/mol und ein massenmittleres Molekulargewicht von M_{w} = 329.000 g/mol auf.

### Copolymer VP/MAM/ImEMA (Polymer10)

Zur Herstellung eines P(VP-co-MAM-co-ImEMA) Copolymers wurde die Vorlage (55,19 g VE-Wasser, 7 g VP, 16,01 g Zulauf 1, 1,04 g Zulauf 2) mit Stickstoff begast und auf eine Reaktorinnentemperatur von 70 °C erhitzt. Nach Erreichen der Innentemperatur wurden Zulauf 1 (266,67 g MAM, 0,35 g Phosphorsäure, 3,19 g ImEMA, 50 g VP) und 2 (0,8 g Wako V50, 20 g VE-Wasser) gestartet. Zulauf 1 wurde in 4 Stunden, Zulauf 2 in 4,5 Stunden zudosiert. Nach Zulaufende wurde noch 2 Stunden weiter polymerisiert. Man erhält eine farblose, leicht opake Polymerlösung. Analog wurden die Polymere 11, 12 und V1 der Tabelle 2 hergestellt.

### Bestimmung des K-Wertes

Die K-Werte wurden nach Fikentscher, Cellulosechemie, Bd. 13, S. 58 bis 64 (1932) bei 25°C in wässriger Lösung gemessen und stellen ein Maß für das Molgewicht dar. Die wässrige Lösung der Polymere enthält 1 g Polymer in 100 ml Lösung. Die Messung des K-Wertes erfolgt in einer Micro-Ubbelohde-Kapillare Typ M Ic der Fa. Schott.

### Klebrigkeit

Die Klebrigkeit der Polymerfilme wurde nach der in Chemie in unserer Zeit 36 (2002) Seiten 44-52 beschriebenen Methode bestimmt.

### Prüfung der Biegefestigkeit:

Die Prüfungen wurden in einem Klimaraum bei 20°C und 65% rel. Feuchte mittels eines Zug/Druck - Prüfgerätes (Typ Easytest 86 802, Fa. Frank) durchgeführt. Die Haarsträhne wurde symmetrisch auf zwei zylindrische Rollen (Durchmesser 4 mm, Abstand = 90 mm) der Probenaufnahme gelegt. Genau in der Mitte wurde die Strähne dann von oben mit einem abgerundetem Stempel um ca. 40 mm durchgebogen (bis zum Brechen des Gelfilmes). Die dafür erforderliche Kraft wurde mit einer Wägezelle (50 N) gemessen und in Newton angegeben.

### Beispiele für kosmetische Zusammensetzungen

Haarkosmetische Zusammensetzung (allg.)
a) 0,01 bis 5 Gew.-% eines erfindungsgemäß verwendeten Polymers
b) 25 bis 99,99 Gew.-% Wasser und/oder Alkohol
c) 0 bis 95,99 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen, Vitamine, Eiweiß-hydrolysate, Alpha- und Beta-Hydroxycarbonsäuren, Chitosan, Eiweißhydrolysate, Kosmetik-Polymere, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

### Shampooformulierung/Duschgelformulierung

Bevorzugte Shampooformulierungen oder Duschgelformulierungen enthalten
a) 0,01 bis 5 Gew.-% eines erfindungsgemäß verwendeten Polymers
b) 25 bis 99,99 Gew.-% Wasser
c) 0 - 5 Gew.-% eines weiteren Konditioniermittels
d) 0 - 30 Gew.-% weitere kosmetische Bestandteile

In den Shampooformulierungen können zudem alle in Shampoos üblicherweise eingesetzten anionischen, neutralen, amphoteren oder kationischen Tenside verwendet werden mit den vorstehenden Maßgaben.

### Beispiel 1: Conditioner Shampoo mit PQ-10

| | |
|---|---|
| 35,70 g | Sodium Laureth Sulfate |
| 6,50 g | Cocamidopropyl Betaine |
| 0,20 g | Polymer 5 |
| 0,40 g | Polyquaternium-10 |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle des Polymers gemäß Beispiel 5 eines oder mehrere der Polymere 1 bis 4 oder 6 bis 12 verwendet werden.

### Beispiel 2: Conditioner Shampoo mit GHTC

| | |
|---|---|
| 35,70 g | Sodium Laureth Sulfate |
| 6,50 g | Cocamidopropyl Betaine |
| 0,50 g | Polymer 5 |
| 0,20 g | Guarhydroxypropyltrimonium Chloride |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle des Polymers gemäß Beispiel 5 eines oder mehrere der Polymere 1 bis 4 oder 6 bis 12 verwendet werden.

### Beispiel 3: Conditioner Shampoo mit Polyquaternium

| | |
|---|---|
| 35,70 g | Sodium Laureth Sulfate |
| 6,50 g | Cocamidopropyl Betaine |
| 0,20 g | Polymer 5 |
| 0,30 g | Polyquaternium-44 oder PQ-67 |
| 0,10 g | Konservierungsmittel |
| 0,10 g | Parfumöl / etherisches Öl |
| ad 100 g | Aqua dem. |

Gute Conditioner Shampoos werden auch erhalten, wenn anstelle des Polymers gemäß Beispiel 5 eines oder mehrere der Polymere 1 bis 4 oder 6 bis 12 verwendet werden.

### Beispiel 4: Shampoo

| Phase A | |
|---|---|
| 15,00 g | Cocamidopropyl Betaine |
| 10,00 | g Disodium Cocoamphodiacetate |
| 5,00 g | Polysorbate 20 |
| 5,00 g | Decyl Glucoside |
| 0,20 g | Polymer 5 |
| 0,10 g | Parfumöl / etherisches Öl |
| q.s. | Konservierungsmittel |
| 2,00 g | Laureth-3 |
| ad 100 | Aqua dem. |
| q.s. | Citric Acid |
| | |

| Phase B | |
|---|---|
| 3,00 g | PEG-150 Distearate |

### Herstellung

Komponenten der Phase A einwiegen und lösen; pH-Wert auf 6-7 einstellen. Phase B zugeben und auf 50°C erwärmen. Auf Raumtemperatur abkühlen lassen unter Rühren.

Gute Shampoos werden auch erhalten, wenn anstelle des Polymers gemäß Beispiel 5 eines oder mehrere der Polymere 1 bis 4 oder 6 bis 12 verwendet werden.

### Beispiel 5: Shampoo

| | |
|---|---|
| 30,00 g | Sodium Laureth Sulfate |
| 6,00 g | Sodium Cocoamphoacetate |
| 0,50 g | Polymer 5 |
| 3,00 g | Sodium Laureth Sulfate, Glycol Distearate, Cocamide MEA, Laureth-10 |
| 2,00 g | Dimethicone |
| q.s. | Parfum |
| q.s. | Konservierungsmittel |
| q.s. | Citric Acid |
| 1,00 g | Sodium Chloride |
| add 100 | Aqua dem. |

Gute Shampoos werden auch erhalten, wenn anstelle des Polymers gemäß Beispiel 5 eines oder mehrere der Polymere 1 bis 4 oder 6 bis 12 verwendet werden.

### Beispiel 6: Duschgel

| | |
|---|---|
| 20,00 g | Ammonium Laureth Sulfate |
| 15,00 g | Ammonium Lauryl Sulfate |
| 0,50 g | Polymer 5 |
| 0,50 g | Polyquaternium-7 |
| 2,50 g | Sodium Laureth Sulfate, Glycol Distearate, Cocamide MEA, Laureth-10 |
| 0,10 g | Parfumöl / etherisches Öl |
| q.s. | Konservierungsmittel |
| 0,50 g | Sodium Chloride |
| add 100 | Aqua dem. |

Gute Duschgele werden auch erhalten, wenn anstelle des Polymers gemäß Beispiel 5 eines oder mehrere der Polymere 1 bis 4 oder 6 bis 12 verwendet werden.

### Beispiel 7: Duschgel

| | |
|---|---|
| 40,00 g | Sodium Laureth Sulfate |
| 5,00 g | Decyl Glucoside |
| 5,00 g | Polymer 5 |
| 1,00 g | Panthenol |
| 0,10 g | Parfumöl / etherisches Öl |
| q.s. | Konservierungsmittel |
| q.s. | Citric Acid |
| 2,00 g | Sodium Chloride |
| add 100 | Aqua dem. |

Gute Duschgele werden auch erhalten, wenn anstelle des Polymers gemäß Beispiel 5 eines oder mehrere der Polymere 1 bis 4 oder 6 bis 12 verwendet werden.

### Beispiel 8: Shampoo

| | |
|---|---|
| 12,00 g | Sodium Laureth Sulfate |
| 1,50 g | Decyl Clucoside |
| 0,50 g | Polymer 5 |
| 5,00 g | Coco-Glucoside Glyceryl Oleate |
| 2,00 g | Sodium Laureth Sulfate, Glycol Distearate, Cocomide MEA, Laureth-10 |
| q.s. | Konservierungsmittel |
| q.s. | Sunset Yelow C. I. 15 985 |
| 0,10 g | Parfumöl / etherisches Öl |
| 1,00 g | Sodium Chloride |
| add 100 | Aqua dem. |

Gute Shampoos werden auch erhalten, wenn anstelle des Polymers gemäß Beispiel 5 eines oder mehrere der Polymere 1 bis 4 oder 6 bis 12 verwendet werden.

Die erfindungsgemäßen Polymere werden auch in Haarstyling-Zubereitungen verwendet, insbesondere in Haarschäumen (Aerosolschäume mit Treibgas und Pumpschäume ohne Treibgas), Haarsprays (Pumpssprays ohne Treibgas) und Haargelen.

Treibmittel sind die üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

### Aerosolhaarschaum

a) 0,1 bis 10 Gew.-% eines Kosmetik-Polymers
b) 55 bis 99,8 Gew.-% Wasser und/oder Alkohol
c) 5 bis 20 Gew.-% eines Treibmittels
d) 0,1 bis 5 Gew.-% eines erfindungsgemäß verwendeten Polymers
e) 0 bis 10 Gew.-% weitere Bestandteile

Als weitere Bestandteile können unter anderem alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethyl-ammoniumdihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

### Beispiel 9: Aerosolhaarschaum

| | |
|---|---|
| 2.00 g | Cocotrimonium Methosulfate |
| 0,10 g | Parfumöl / etherisches Öl |
| 3,50 g | Festigerpolymer z.B. Polyquaternium-46, PQ-44, VP/Methacrylamide/ Vinyl Imidazole Copolymer, etc. |
| 0,80 g | Polymer 3 |
| q.s. | Preservative |
| 75,00 g | Wasser dem. |
| 10,00 g | Propane/Butane (3.5 bar) |

Gute Aerosolhaarschäume werden auch erhalten, wenn anstelle des Polymers gemäß Beispiel 3 eines oder mehrere der Polymere 1, 2 oder 4 bis 12 verwendet werden.

### Stylinggel

a) 0,1 bis 10 Gew.-% eines Kosmetik-Polymers
b) 60 bis 99,85 Gew.-% Wasser und/oder Alkohol
c) 0,05 bis 10 Gew.-% eins Gelbildners
d) 0,1 bis 5 Gew.-% eines erfindungsgemäßen Polymers
e) 0 bis 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium-37 (and) Propylene Glycole Dicaprate Dicaprylate (and) PPG-1 Trideceth-6, Polyquaternium-7, Polyquaternium-44, Polyquaternium-67.

Gute Stylinggele werden auch erhalten, wenn anstelle des Polymers gemäß Beispiel 3 eines oder mehrere der Polymere 1, 2 oder 4 bis 12 verwendet werden.

### Beispiel 10: Haarstylinggel

| Phase A | |
|---|---|
| 0,50 g | Carbomer oder Acrylates/C10-30 Alkyl Acrylate Crosspolymer |
| 86,40 g | Wasser dem. |
| | |

| Phase B | |
|---|---|
| 0,70 g | Triethanolamine |
| | |

| Phase C | |
|---|---|
| 6,00 g | Festigerpolymer z.B. VP/Methacrylamide/Vinyl Imidazole Copolymer |
| 5,00g | PVP |
| 0,20 g | PEG-25 PABA |
| 0,50 g | Polymer 3 |
| 0,10 g | Parfumöl / etherisches Öl |
| q.s. | PEG-14 Dimethicone |
| q.s. | Konservierungsmittel |
| 0,10 g | Tocopheryl Acetate |

Gute Stylinggele werden auch erhalten, wenn anstelle des Polymers gemäß Beispiel 3 eines oder mehrere der Polymere 1, 2 oder 4 bis 12 verwendet werden.

### Beispiel 11: Haarstylinggel

| Phase A | |
|---|---|
| 0,50 g | Carbomer oder Acrylates/C10-30 Alkyl Acrylate Crosspolymer |
| 91,20 g | Wasser dem. |
| | |

| Phase B | |
|---|---|
| 0,90 g | Tetrahydroxypropyl Ethylenediamine |
| | |

| Phase C | |
|---|---|
| 7,00 g | VP/VA Copolymer |
| 0,40 g | Polymer 3 |
| 0,20 g | Parfumöl / etherisches Öl |
| q.s. | Konservierungsmittel |
| 0,10 g | Propylene Glycol |

Gute Stylinggele werden auch erhalten, wenn anstelle des Polymers gemäß Beispiel 3 eines oder mehrere der Polymere 1, 2 oder 4 bis 12 verwendet werden.

### Beispiel 12: Hair Wax Cream

| | |
|---|---|
| 6,00 g | Caprylic/Capric Triglyceride |
| 3,00 g | Glyceryl Stearate |
| 2,00 g | Cetyl Alcohol |
| 3,50 g | Polymer 3 |
| 0,50 g | Cremophoer A6 |
| 0,70 g | Cremophor A25 |
| 0,50 g | Dimethicone |
| 0,50 g | Vitamin E Acetate |
| 2,00 g | Caprylic/Capric Triglyceride and Sodiumacrylates Copolymer |
| 1,00 g | D-Panthenol USP |
| 0,10 g | EDTA |
| 10,00 g | Festigerpolymer |
| q.s. | Konservierungsmittel |
| ad 100 g | Wasser dem. |

Gute Hair Wax Creams werden auch erhalten, wenn anstelle des Polymers gemäß Beispiel 3 eines oder mehrere der Polymere 1, 2 oder 4 bis 12 verwendet werden.

### Beispiel 13: Haarpudding

| | |
|---|---|
| 3,00 g | Kollicoat IR (BASF) |
| q.s. | Konservierungsmittel |
| 2,00 g | Festigerpolymer |
| 4,00 g | Acrylates/beheneth-25 Methacrylate Copolymer |
| 0,70 g | Polymer 3 |
| 0,50 g | Dimethicone Copolyol |
| 0,10 g | EDTA |
| 0,20 g | Benzophenone-4 |
| ad 100 g | Wasser dem. |

Gute Haarpuddings werden auch erhalten, wenn anstelle des Polymers gemäß Beispiel 3 eines oder mehrere der Polymere 1, 2 oder 4 bis 12 verwendet werden.

### Beispiel 14: Sprühgel

| Phase A | |
|---|---|
| 1,25 g | Festigerpolymer |
| 96,15 g | Aqua dem. |
| | |

| Phase B | |
|---|---|
| 0,70 g | Acrylates/Steareth-20 Itaconate Copolymer |
| 0,10 g | Propylene Glycol |
| 0,50 g | Polymer 3 |
| 0,10 g | Glycerin |
| 0,10 g | Parfumöl / etherisches Öl |
| q.s. | Konservierungsmittel |
| | |

| Phase C | |
|---|---|
| 0,70 g | Triethanolamine |

Gute Sprühgele werden auch erhalten, wenn anstelle des Polymers gemäß Beispiel 3 eines oder mehrere der Polymere 1, 2 oder 4 bis 12 verwendet werden.

Eine erfindungsgemäß für Styling-Sprays geeignete Zubereitung kann beispielsweise wie folgt zusammengesetzt sein:

### Beispiel 15: Pumphaarspray

| | |
|---|---|
| 11,20 g | PEG/PPG-25/25 Dimethicone/Acrylates Copolymer |
| 2,80 g | VP/VA Copolymer |
| 1,34 g | Aminomethyl Propanol |
| 0,30 g | Polymer 3 |
| 0,10 g | Parfumöl / etherisches Öl |
| 11,26 g | Aqua dem. |
| 73,00 g | Alcohol |

Gute Pumphaarsprays werden auch erhalten, wenn anstelle des Polymers gemäß Beispiel 3 eines oder mehrere der Polymere 1, 2 oder 4 bis 12 verwendet werden.

### Beispiel 16: Pumphaarspray VOC55

| | |
|---|---|
| 2,00 g | VP/Methacrylamide/ Vinyl Imidazole Copolymer |
| 1,90 g | Polyquaternium-46 |
| 2,00 g | Polymer 3 |
| 0,10 g | Parfumöl / etherisches Öl |
| 55,00 g | Alcohol |
| 39,00 g | Aqua dem. |

Gute Pumphaarsprays VOC 55 werden auch erhalten, wenn anstelle des Polymers gemäß Beispiel 3 eines oder mehrere der Polymere 1, 2 oder 4 bis 12 verwendet werden.

Hautkosmetische Zusammensetzungen

### Beispiel 17: Flüssiges Makeup

| Phase A | |
|---|---|
| 1,70 g | Glyceryl Stearate |
| 1,70 g | Cetyl Alcohol |
| 1,70 g | Ceteareth-6 |
| 1,70 g | Ceteareth-25 |
| 5,20 g | Caprylic/Capric Triglyceride |
| 5,20 g | Mineral Oil oder Luvitol^{®} Lite (INCI Hydrogenated Polyisobuten) |
| | |

| Phase B | |
|---|---|
| q.s. | Konservierungsmittel |
| 4,30 g | Propylene Glycol |
| 2,50 g | Polymer 3 |
| 59,50 g | Aqua dem. |
| | |

| Phase C | |
|---|---|
| 0,10 g | Parfumöl / etherisches Öl |
| | |

| Phase D | |
|---|---|
| 2,00 g | Iron Oxides |
| 12,00 g | Titanium Dioxide |

Gute Flüssig-Make-ups werden auch erhalten, wenn anstelle des Polymers gemäß Beispiel 3 eines oder mehrere der Polymere 1, 2 oder 4 bis 12 verwendet werden.

### Beispiel 18: Eyeliner

| Phase A | |
|---|---|
| 40,60 g | dest. Wasser |
| 0,20 g | Disodium EDTA |
| q.s. | Konservierungsmittel |
| | |

| Phase B | |
|---|---|
| 0,60 g | Xanthan Gum |
| 0,40 g | Veegum |
| 3,00 g | Butylene Glycol |
| 0,20 g | Polysorbate-20 |
| | |

| Phase C | |
|---|---|
| 15,00 g | Iron oxide /Al Powder / Silica (z.B. Sicopearl^{®} Fantastico Gold von BASF) |
| | |

| Phase D | |
|---|---|
| 10,00 g | Aqua dem. |
| 25,00 g | Festigerpolymer (z.B. Polyurethane-1 oder VP/Methacrylamide/ Vinyl Imidazole Copolymer, etc.) |
| 5,00 g | Polymer 3 |

Gute Eyeliner werden auch erhalten, wenn anstelle des Polymers gemäß Beispiel 3 eines oder mehrere der Polymere 1, 2 oder 4 bis 12 verwendet werden.

### Beispiel 19: Sonnenschutz-Gel

| Phase A | |
|---|---|
| 0,90 g | Polymer 3 |
| 8,00 g | Octyl Methoxycinnamate |
| 5,00 g | Octocrylene |
| 0,80 g | Octyl Triazone |
| 2,00 g | Butyl Methoxydibenzoylmethane |
| 2,00 g | Tocopheryl Acetate |
| 0,10 g | Parfumöl / etherisches Öl |
| | |

| Phase B | |
|---|---|
| 0,30 g | Acrylates/C10-30 Alkyl Acrylate Crosspolymer |
| 0,20 g | Carbomer |
| 5,00 g | Glycerin |
| 0,20 g | Disodium EDTA |
| q.s. | Konservierungsmittel |
| 75,30 g | Aqua dem. |

| Phase C | |
|---|---|
| 0,20 g | Sodium Hydroxide |

Gute Sonnenschutzgele werden auch erhalten, wenn anstelle des Polymers gemäß Beispiel 3 eines oder mehrere der Polymere 1, 2 oder 4 bis 12 verwendet werden.

### Beispiel 20: Sonnenschutzemulsion mit TiO₂ und ZnO₂

| Phase A | |
|---|---|
| 1,00 g | PEG-7 Hydrogenated Castor Oil |
| 5,00 g | Polymer 3 |
| 2,00 g | PEG-45/Dodecyl Glycol Copolymer |
| 3,00 g | Isopropyl Myristate |
| 7,90 g | Jojoba (Buxus Chinensis) Oil |
| 4,00 g | Octyl Methoxycinnamate |
| 2,00 g | 4-Methylbenzylidene Camphor |
| 3,00 g | Titanium Dioxide, Dimethicone |
| 1,00 g | Dimethicone |
| 5,00 g | Zinc Oxide, Dimethicone |
| | |

| Phase B | |
|---|---|
| 0,20 g | Disodium EDTA |
| 5,00 g | Glycerin |
| q.s. | Konservierungsmittel |
| 60,80 g | Aqua dem. |
| | |

| Phase C | |
|---|---|
| 0,10 g | Parfumöl / etherisches Öl |

Gute Sonnenschutzemulsionen werden auch erhalten, wenn anstelle des Polymers gemäß Beispiel 3 eines oder mehrere der Polymere 1, 2 oder 4 bis 12 verwendet werden.

### Beispiel 21: Gesichtswasser

| Phase A | |
|---|---|
| 3,00 g | Polymer 3 |
| 0,10 g | Parfumöl / etherisches Öl |
| 0,30 g | Bisabolol |

| Phase B | |
|---|---|
| 3,00 g | Glycerin |
| 1,00 g | Hydroxyethyl Cetyldimonium Phosphate |
| 5,00 g | Whitch Hazel (Hamamelis Virginiana) Distillate |
| 0,50 g | Panthenol |
| q.s. | Konservierungsmittel |
| 87,60 g | Aqua dem. |

Gute Gesichtswässer werden auch erhalten, wenn anstelle des Polymers gemäß Beispiel 3 eines oder mehrere der Polymere 1, 2 oder 4 bis 12 verwendet werden.

### Beispiel 22: Gesichtswaschpaste mit Peelingeffekt

| Phase A | |
|---|---|
| 73,00 g | Aqua dem. |
| 1,50 g | Carbomer |
| q.s. | Konservierungsmittel |
| | |

| Phase B | |
|---|---|
| q.s. | Parfümöl |
| 7,00 g | Potassium Cocoyl Hydrolyzed Protein |
| 4,00 g | Polymer 3 |
| | |

| Phase C | |
|---|---|
| 1,50 g | Triethanolamine |
| | |

| Phase D | |
|---|---|
| 13,00 g | Polyethylene (Luwax A™ von BASF) |

Gute Gesichtswaschpasten werden auch erhalten, wenn anstelle des Polymers gemäß Beispiel 3 eines oder mehrere der Polymere 1, 2 oder 4 bis 12 verwendet werden.

### Beispiel 23: Seife

| Phase A | |
|---|---|
| 25,00 g | Potassium Cocoate |
| 20,00 g | Disodium Cocoamphodiacetate |
| 2,00 g | Lauramide DEA |
| 1,0 g | Glycol Stearate |
| 2,00 g | Polymer 3 |
| 50,00 g | Aqua dem. |
| q.s. | Citric Acid |
| | |

| Phase B | |
|---|---|
| q.s. | Konservierungsmittel |
| 0,10 g | Parfumöl / etherisches Öl |

Gute Seifen werden auch erhalten, wenn anstelle des Polymers gemäß Beispiel 3 eines oder mehrere der Polymere 1, 2 oder 4 bis 12 verwendet werden.

### Beispiel 24: Gesichtsreinigungsmilch Typ O/W

| Phase A | |
|---|---|
| 1,50 g | Ceteareth-6 |
| 1,50 g | Ceteareth-25 |
| 2,00 g | Glyceryl Stearate |
| 2,00 g | Cetyl Alcohol |
| 10,00 g | Mineral Oil |
| | |

| Phase B | |
|---|---|
| 5,00 g | Propylene Glycol |
| q.s. | Konservierungsmittel |
| 1,00 g | Polymer 3 |
| 66,30 g | Aqua dem. |
| | |

| Phase C | |
|---|---|
| 0,20 g | Carbomer |
| 10,00 g | Cetearyl Octanoate |
| | |

| Phase D | |
|---|---|
| 0,40 g | Tetrahydroxypropyl Ethylenediamine |
| | |

| Phase E | |
|---|---|
| 0,10 g | Parfumöl / etherisches Öl |
| 0,10 g | Bisabolol |

Gute Gesichtsreinigungsmilche werden auch erhalten, wenn anstelle des Polymers gemäß Beispiel 3 eines oder mehrere der Polymere 1, 2 oder 4 bis 12 verwendet werden.

### Beispiel 25: Transparente Seife

| | |
|---|---|
| 4,20 g | Sodium Hydroxide |
| 3,60 g | dest. Wasser |
| 10,00 g | Polymer 3 |
| 22,60 g | Propylene Glycol |
| 18,70 g | Glycerin |
| 5,20 g | Cocoamide DEA |
| 2,40 g | Cocamine Oxide |
| 4,20 g | Sodium Lauryl Sulfate |
| 7,30 g | Myristic Acid |
| 16,60 g | Stearic Acid |
| 5,20 g | Tocopherol |

Gute transparente Seifen werden auch erhalten, wenn anstelle des Polymers gemäß Beispiel 3 eines oder mehrere der Polymere 1, 2 oder 4 bis 12 verwendet werden.

### Beispiel 26: Rasierschaum

| | |
|---|---|
| 6,00 g | Ceteareth-25 |
| 5,00 g | Poloxamer 407 |
| 52,00 g | Aqua dem. |
| 1,00 g | Triethanolamine |
| 5,00 g | Propylene Glycol |
| 1,00 g | PEG-75 Lanolin Oil |
| 5,00 g | Polymer 3 |
| q.s. | Konservierungsmittel |
| 0,10 g | Parfumöl / etherisches Öl |
| 25,00 g | Sodium Laureth Sulfate |

Abfüllung: 90 Teile Wirksubstanz und 10 Teile Propan/Butan-Mischung 25:75.

Gute Rasierschäume werden auch erhalten, wenn anstelle des Polymers gemäß Beispiel 3 eines oder mehrere der Polymere 1, 2 oder 4 bis 12 verwendet werden.

### Beispiel 27: After Shave Balsam

| Phase A | |
|---|---|
| 0,25 g | Acrylates/C10-30 Alkyl Acrylate Crosspolymer |
| 1,50 g | Tocopheryl Acetate |
| 0,20 g | Bisabolol |
| 10,00 g | Caprylic/Capric Triglyceride |
| q.s. | Parfum |
| 1,00 g | Polymer 3 |
| | |

| Phase B | |
|---|---|
| 1,00 g | Panthenol |
| 15,00 g | Alcohol |
| 5,00 g | Glycerin |
| 0,05 g | Hydroxyethyl Cellulose |
| 1,90 g | Polymer 3 |
| 64,02 g | dest. Wasser |
| | |

| Phase C | |
|---|---|
| 0,08 g | Sodium Hydroxide |

Gute After-Shave-Balsams werden auch erhalten, wenn anstelle des Polymers gemäß Beispiel 3 eines oder mehrere der Polymere 1, 2 oder 4 bis 12 verwendet werden.

### Beispiel 28: Pflegecreme

| Phase A | |
|---|---|
| 2,00 g | Ceteareth-6 |
| 2,00 g | Ceteareth-25 |
| 2,00 g | Cetearyl Alcohol |
| 3,00 g | Glyceryl Stearate SE |
| 5,00 g | Mineral Oil |
| 4,00 g | Jojoba (Buxus Chinensis) Oil |
| 3,00 g | Cetearyl Octanoate |
| 1,00 g | Dimethicone |
| 3,00 g | Mineral Oil, Lanolin Alcohol |
| | |

| Phase B | |
|---|---|
| 5,00 g | Propylene Glycol |
| 0,50 g | Veegum |
| 1,00 g | Panthenol |
| 1,70 g | Polymer 3 |
| 6,00 g | Polyquaternium-44 |
| q.s. | Konservierungsmittel |
| 60,80 g | Auqa dem. |
| | |

| Phase C | |
|---|---|
| q.s. | Parfum |

Gute Pflegecremes werden auch erhalten, wenn anstelle des Polymers gemäß Beispiel 3 eines oder mehrere der Polymere 1, 2 oder 4 bis 12 verwendet werden.

Mund- und Zahnpflegezubereitungen

### Beispiel 29: Zahnpaste

| Phase A | |
|---|---|
| 34,79 g | Aqua dem. |
| 3,00 g | Polymer 3 |
| 20,00 g | Glycerin |
| 0,76 g | Sodium Monofluorophosphate |
| | |

| Phase B | |
|---|---|
| 1,20 g | Sodium Carboxymethylcellulose |
| | |

| Phase C | |
|---|---|
| 0,80 g | Aromaöl |
| 0,06 g | Saccharin |
| q.s. | Konservierungsmittel |
| 0,05 g | Bisabolol |
| 1,00 g | Panthenol |
| 0,50 g | Tocopheryl Acetate |
| 2,80 g | Silica |
| 1,00 g | Sodium Lauryl Sulfate |
| 7,90 g | Dicalciumphosphate Anhydrate |
| 25,29 g | Dicalciumphosphate Dihydrate |
| 0,45 g | Titanium Dioxide |

Gute Zahnpasten werden auch erhalten, wenn anstelle des Polymers gemäß Beispiel 3 eines oder mehrere der Polymere 1, 2 oder 4 bis 12 verwendet werden.

### Beispiel 30: Mundwasser

| Phase A | |
|---|---|
| 2,00 g | Aromaöl |
| 4,50 g | Polymer 3 |
| 1,00 g | Bisabolol |
| 30,00 g | Alcohol |
| | |

| Phase B | |
|---|---|
| 0,20 g | Saccharin |
| 5,00 g | Glycerin |
| q.s. | Konservierungsmittel |
| 5,00 g | Poloxamer 407 |
| 52,30 g | Aqua dem. |

Gute Mundwässer werden auch erhalten, wenn anstelle des Polymers gemäß Beispiel 3 eines oder mehrere der Polymere 1, 2 oder 4 bis 12 verwendet werden.

### Beispiel 37: Prothesenhaftmittel

| Phase A | |
|---|---|
| 0,20 g | Bisabolol |
| 1,00 g | Beta-Carotene |
| q.s. | Aromaöl |
| 20,00 g | Cetearyl Octanoate |
| 5,00 g | Silica |
| 33,80 g | Mineral Oil |
| | |

| Phase B | |
|---|---|
| 5,00 g | Polymer 3 |
| 35,00 g | PVP (20%ige Lösung in Wasser) |

Gute Prothesenhaftmittel werden auch erhalten, wenn anstelle des Polymers gemäß Beispiel 3 eines oder mehrere der Polymere 1, 2 oder 4 bis 12 verwendet werden.

### Beispiel 38: Flüssige Seife

| | |
|---|---|
| 15,0 g | Kokosfettsäure, Kaliumsalz |
| 3,0 g | Kaliumoleat |
| 5,0 g | Luvitol®Lite (BASF) |
| 2,0 g | Polymer Vinylpyrrolidon/Stearylmethacrylat 70/30 Gew.-% (K-Wert 47; 1 % in Isopropanol) |
| 1,0 g | Glycerinstearat |
| 0,5 g | Polymer 3 |
| 2,0 g | Ethylenglycoldistearat |
| ad 100 | Spezifische Zusätze, Komplexierungsmittel, Duftstoffe, Wasser |

Gute Flüssigseifen werden auch erhalten, wenn anstelle des Polymers gemäß Beispiel 3 eines oder mehrere der Polymere 1, 2 oder 4 bis 12 verwendet werden.

### Beispiele 39-41: Conditioner Shampoo mit Perlglanz

| Angaben in Gew.-% | | | |
|---|---|---|---|
| Zusatz | Bsp. 39 | Bsp. 40 | Bsp. 41 |
| Polymer 5 | 0,5 | 0,5 | 0,5 |
| Natriumlaurethsulfat | 9,0 | 9,0 | 9,0 |
| Cocoamidopropylbetain | 2,5 | 2,5 | 2,5 |
| Benzophenon-3 | 1,5 | 0,5 | 1,00 |
| Perlglanzmittel | 2,0 | 2,0 | 2,0 |
| Luvitol Lite^{®}(BASF) | 0,1 | 0,15 | 0,05 |
| Dinatrium EDTA | 0,1 | 0,2 | 0,15 |
| Konvervierungsmittel, Parfüm, Verdicker, pH-Einstellung und Lösungsvermittler | q.s. | q.s. | q.s. |
| Wasser | ad 100,0 | ad 100,0 | ad 100,0 |
| Der pH-Wert wird auf 6 eingestellt. | | | |

Gute Conditionier Shampoos mit Perlglanz werden auch erhalten, wenn anstelle des Polymers gemäß Beispiel 5 eines oder mehrere der Polymere 1 bis 4 oder 6 bis 12 verwendet werden.

### Beispiele 42-46: Formulierungen zum Duschen, Waschen, Baden

| Angaben in Gew.-% | | | | | |
|---|---|---|---|---|---|
| Zusatz | Bsp. 42 | Bsp. 43 | Bsp. 44 | Bsp. 45 | Bsp. 46 |
| Texapon N 70 | 13,00 | 15,00 | 10,50 | 12,50 | 10,00 |
| Dehyton PK 45 | 7,50 | 7,00 | 5,00 | 5,50 | 10,00 |
| Cetiol HE | 2,00 | 2,50 | 3,50 | 5,00 | 2,30 |
| Parfüm | 0,10 | 0,10 | 0,10 | 0,10 | 0,10 |
| Luvitol^{®}Lite (BASF) | 1,00 | 4,50 | 7,00 | 1,40 | 3,00 |

| Zusatz | Bsp. 42 | Bsp. 43 | Bsp. 44 | Bsp. 45 | Bsp. 46 |
|---|---|---|---|---|---|
| D-Panthenol USP | 1,00 | 1,50 | 1,80 | 1,70 | 1,40 |
| Konservierungsmittel | 0,10 | 0,10 | 0,10 | 0,10 | 0,10 |
| Zitronensäure | 0,10 | 0,10 | 0,10 | 0,10 | 0,10 |
| Polymer 3 | 0,50 | 1,00 | 0,50 | 0,20 | 0,10 |
| Natriumchlorid | 1,50 | 1,40 | 1,40 | 1,30 | 1,50 |
| Wasser dem. | ad 100 | ad 100 | ad 100 | ad 100 | ad 100 |

Gute Formulierungen zum Duschen, Waschen, Baden werden auch erhalten, wenn anstelle des Polymers gemäß Beispiel 3 eines oder mehrere der Polymere 1, 2 oder 4 bis 12 verwendet werden.

## Patentansprüche

1. Verwendung von Polymeren, die wenigstens eine Verbindung der allgemeinen Formel (I) und/oder ihrer kationischen Form (la) einpolymerisiert enthalten 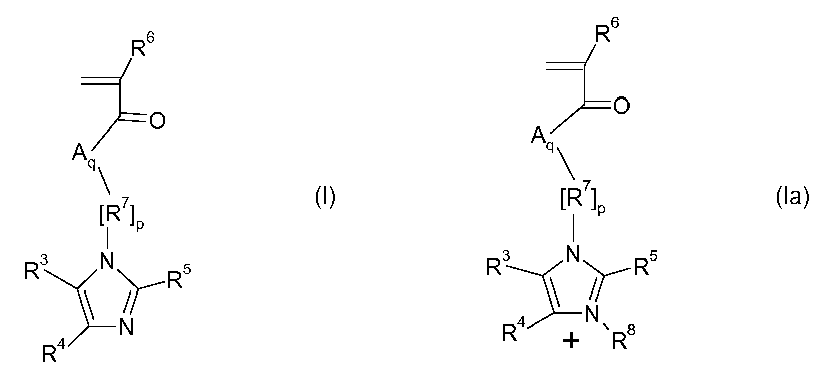 wobei
R³, R⁴ und R⁵ unabhängig voneinander H oder C₁-C₂₀-Alkyl, C₁-C₂₀-Alkylcarbonyl, C₂-C₂₀-Alkenyl, C₂-C₂₀-Alkenylcarbonyl, C₂-C₂₀-Alkinyl, C₂-C₂₀-Alkinylcarbonyl, C₃-C₁₅-Cycloalkyl, C₃-C₁₅-Cycloalkylcarbonyl, Aryl, Arylcarbonyl, ein Heterocyclus oder ein Halogenatom,
R⁶ H oder Methyl,
R⁷ zweiwertiger organischer Rest,
R⁸ H oder C₁-C₁₀-Alkyl,
A O oder NH,
p, q unabhängig voneinander 0 oder 1 bedeuten,
in kosmetischen Zubereitungen.

2. Verwendung nach Anspruch 1, wobei die Verbindung der allgemeinen Formel (I) 2-(1-Imidazolyl)ethylmethacrylat und/oder die Verbindung der allgemeinen Formel (la) am Stickstoff quaternisiertes 2-(1-Imidazolyl)ethylmethacrylat ist.

3. Verwendung nach einem der Ansprüche 1 oder 2, wobei die Polymere wenigstens ein weiteres Monomer einpolymerisiert enthalten.

4. Verwendung nach Anspruch 3, wobei das wenigstens eine weitere Monomer ausgewählt ist aus N-Vinyllactamen, Säuregruppen enthaltenden Monomeren, (Meth)acrylsäureestern, (Meth)acrylsäureamiden, Vinylethern und Diallylaminen.

5. Verwendung nach einem der Ansprüche 3 oder 4, wobei das wenigstens eine weitere Monomer ausgewählt ist aus N-Vinylpyrrolidon, (Meth)acrylsäure, Methylmethacrylat, N,N-Diallyl-N,N-Dimethylammoniumchlorid, N,N-Dimethylaminoethylmethacrylat, N-[3-(dimethylamino)propyl]methacrylamid, Methacrylamid und deren Mischungen.

6. Verwendung nach einem der Ansprüche 1 bis 5, wobei die Polymere wenigstens zwei weitere Monomere einpolymerisiert enthalten, wobei wenigstens eines der weiteren Monomere ausgewählt ist aus N-Vinyllactamen und wenigstens eines der weiteren Monomere ausgewählt ist aus (Meth)acrylsäureamiden.

7. Verwendung nach einem der Ansprüche 1 bis 6 als Konditioniermittel.

8. Verwendung nach einem der Ansprüche 1 bis 7 als Haarfestigungsmittel.

9. Verwendung nach einem der Ansprüche 1 bis 8 als Verdickungsmittel.

10. Kosmetische Zusammensetzung enthaltend wenigstens ein Polymer wie in einem der Ansprüche 1 bis 6 definiert.
